(19) Europäisches Patentamt
European Patent Office
Office européen des brevets

(11) Veröffentlichungsnummer: **0 115 345**
**B1**

(12) # EUROPÄISCHE PATENTSCHRIFT

(45) Veröffentlichungstag der Patentschrift: **07.01.88**

(51) Int. Cl.⁴: **C 07 D 209/52**, C 07 D 209/42, C 07 D 209/54, C 07 B 57/00

(21) Anmeldenummer: **84100858.4**

(22) Anmeldetag: **27.01.84**

(54) **Verfahren zur Racemattrennung optisch aktiver bicyclischer Imino-alpha-carbonsäureester und Verwendung der so erhältlichen Verbindungen zur Synthese von Carboxyalkyldipeptiden.**

(30) Priorität: **31.01.83 DE 3303112**
**31.01.83 DE 3303139**

(43) Veröffentlichungstag der Anmeldung:
**08.08.84 Patentblatt 84/32**

(45) Bekanntmachung des Hinweises auf die Patenterteilung:
**07.01.88 Patentblatt 88/1**

(84) Benannte Vertragsstaaten:
**AT BE CH DE FR GB IT LI LU NL SE**

(56) Entgegenhaltungen:
**EP - A - 0 037 231**
**EP - A - 0 049 658**
**EP - A - 0 050 850**

**QUARTERLY REVIEWS, Band 25, 1971, London, P.H. BOYLE "Methods of Optical Resolution", Seiten 323-341**
**CHEMISCHE BERICHTE, 86. Jahrgang, 1953, Verlag Chemie GmbH Weinheim/BergstrBe, W.LANGENBECK et al. "Racemat-Spaltung des d,1-Phenylalanins, d,1-Valins und d,1-Alanins über ihre Ester", Seiten 1524-1528**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Geiger, Rolf, Prof. Dr., Heinrich-Bleicher-Strasse 33, D-6000 Frankfurt am Main 50 (DE)**
Erfinder: **Teetz, Volker, Dr., An der Tann 20, D-6238 Hofheim am Taunus (DE)**
Erfinder: **Langner, Dietrich, Hansaallee 109, D-6000 Frankfurt am Main (DE)**
Erfinder: **Urbach, Hansjörg, Dr., Le Lavandoustrasse 41, D-6242 Kronberg/Taunus (DE)**
Erfinder: **Henning, Rainer, Dr., Rotenhofstrasse 31, D-6234 Hattersheim am Main (DE)**

EP 0 115 345 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Racemattrennung von Aminosäuren über Kristallisation diastereoisomerer Salze ist ein vielbenütztes Verfahren (Übersicht: Boyle, Quart. Rev. 25 (1971) 323). Meist setzt man N-acylierte Aminosäuren ein, kristallisiert die Salze mit Alkaloidbasen und zerlegt die einheitlichen diastereoisomeren Salze, z.B. durch Extraktion der N-Acylaminosäuren aus der angesäuerten Lösung (J. Amer. Chem. Soc. 71 (1949) 2541, 3251). Man kann auch umgekehrt verfahren und Aminosäureester oder -amide mit optisch aktiven Säuren kristallisieren (Chem. Ber. 86 (1953) 1524).

Man verwendet dazu z.B. optisch aktive Verbindungen wie 10-Camphersulfonsäure, Abietinsäure oder Weinsäure oder deren O-Derivate. Diese Verfahrensweise ist vor allem dann angebracht, wenn optisch aktive Aminosäureester als Ausgangsverbindungen für weitere Synthesen eingesetzt werden sollen. In diesem Fall ist es unvorteilhaft, zuerst eine N-Acylverbindung herzustellen, dann die Racemattrennung über Salzbildung mit optisch aktiven Basen vorzunehmen, den Acylrest abzuspalten und anschliessend die freie Aminosäure zu verestern.

Für bicyclische Imino-α-carbonsäureester ist ein geeignetes Verfahren bisher nicht beschrieben worden. Die experimentelle Prüfung ergab, dass alle üblichen Säuren für die Racemattrennung ungeeignet sind. Für Octahydroindol-2-carbonsäure ist aus der EP-A-37 231 ein Verfahren bekannt, nach der die N-Benzoylverbindung des Racemats über das Salz mit optisch aktivem α-Phenylethylamin getrennt werden kann. Aus den erwähnten Gründen ist dieses Verfahren jedoch unökonomisch, wenn als Zwischenprodukt für weitere Synthesen die Ester benötigt werden.

Es wurde nun gefunden, dass als chirale Partner für die bicyclischen Imino-α-carbonsäureester überraschenderweise N-Acyl-Derivate optisch aktiver R- oder S-Aminosäuren, die einen Phenylkern enthalten, wie z.B. S-Phenylalanin, Tyrosin oder Tyrosin-O-derivate geeignet sind. Dabei fallen aus geeigneten Lösungsmitteln meist die (S, S)- oder (R, R)-Salze spontan aus, während (S, R)- und (R, S)-Salze in Lösung bleiben. Bereits in einem einzigen Schritt kann eine mehr als 95-prozentige Anreicherung erreicht werden, und einmaliges Umkristallisieren führt in hoher Ausbeute zu den optisch einheitlichen Salzen, die in bekannter Weise zerlegt werden.

Die Erfindung betrifft somit ein Verfahren zur Auftrennung racemischer Gemische bicyclischer Imino-α-carbonsäureester in die Komponenten der Formeln Ia und Ib

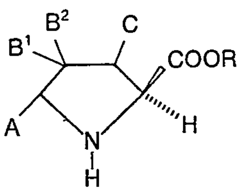

(Ia)

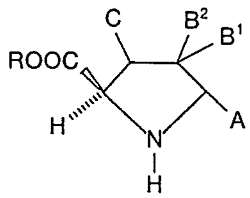

(Ib)

in welchen

R für einen aliphatischen Rest mit 1 bis 6 C-Atomen, einen alicyclischen Rest mit 4 bis 10 C-Atomen, einen aromatischen Rest mit 6 bis 12 C-Atomen oder einen araliphatischen Rest mit 7 bis 15 C-Atomen steht,

a) A und $B^1$ = Wasserstoff bedeuten und $B^2$ und C gemeinsam eine Kette der Formel $-[CH_2]_n$ mit n = 3, 4, 5 oder 6 oder eine Kette der Formel $-[CH_2]_p-CH=CH-[CH_2]_q-$ mit (p+q) = 1, 2, 3 oder 4 bilden,

b) C und $B^2$ = Wasserstoff bedeuten und A und $B^1$ gemeinsam eine Kette der Formel $[CH_2]_n-$ mit n = 3, 4, 5 oder 6 oder eine Kette der Formel $-[CH_2]_p-CH=CH-[CH_2]_q-$ mit (p+q) = 1, 2, 3 oder 4 bilden oder

c) A und C = Wasserstoff bedeuten und $B^1$ und $B^2$ gemeinsam eine Kette der Formel $-[CH_2]_m-$ mit m = 4, 5, 6 oder 7 bilden,

durch Kristallisation diastereomerer Salze, das dadurch gekennzeichnet ist, dass man die Salze der racemischen Ester mit N-acylierten optisch aktiven R- oder S-Aminocarbonsäuren, die einen Phenylkern enthalten und deren eventuell vorhandenen freien OH-Gruppen gegebenenfalls durch Alkyl mit 1 bis 6 C-Atomen, Benzyl oder andere in der Peptidchemie übliche OH-Schutzgruppen O-alkyliert sind, aus der Reihe Phenylalanin, C-Phenylglycin, β-Phenyl-α-amino-buttersäure, 3,4-Dihydroxyphenylalanin, β-Phenylserin und Tyrosin, herstellt, diese aus einem aprotischen organischen Lösemittel oder einem Alkohol mit bis zu 6 C-Atomen umkristallisiert, ausgefallene, optisch einheitliche diastereomere Salze in an sich bekannter Weise zerlegt und die Enantiomeren der Formeln Ia bzw. Ib isoliert und diese gegebenenfalls durch Verseifung bzw. Hydrogenolyse in an sich bekannter Weise in die freien Säuren überführt.

Bevorzugt ist die Racemattrennung von Verbindungen der Formeln Ia bzw. Ib, in welchen

a) A und $B^1$ = Wasserstoff bedeuten und $B^2$ und C gemeinsam eine Kette der Formel $-[CH_2]_n-$ mit n = 3, 4, 5 oder 6 oder eine Kette der Formel $-[CH_2]_p-CH=CH-[CH_2]_q-$ mit (p+q) = 1, 2, 3 oder 4 bilden oder

b) C und $B^2$ = Wasserstoff bedeuten und A und $B^1$ gemeinsam eine der vorstehend unter a) definierten Ketten bilden.

Eine besonders bevorzugte Verfahrensvariante ist dadurch gekennzeichnet, dass man die Salze racemischer bicyclischer Ester der Formeln Ia bzw. Ib, in welchen die beiden Brückenkopf-Wasserstoffatome cis-konfiguriert sind und die COOR-Gruppe endo-ständig zum bicyclischen Ringsystem orientiert ist, vorzugsweise kristallin abscheidet.

Als Imino-α-carbonsäureester kommen vor allem hydrogenolytisch oder hydrolytisch spaltbare Ester mit aliphatischen, alicyclischen oder araliphatischen Alkoholen in Frage, wie sie beispielsweise in Houben-Weyl, Methoden der organischen Chemie, Bd. XV/1, Stuttgart 1974, auf Seite 314–427 oder von Bodanszky et al. in «Peptide Synthesis», 2. Auflage (1976), John Wiley & Sons

beschrieben sind. Bevorzugt sind Ester der Formel Ia + Ib, in denen R für Alkyl mit 1 bis 6 C-Atomen, Cycloalkyl mit 4 bis 8 C-Atomen oder Aralkyl mit 7 bis 13 C-Atomen, das gegebenenfalls durch $NO_2$ substituiert sein kann, steht, insbesondere Alkylester mit bis zu 4 Alkyl-C-Atomen und Aralkylester wie Benzyl-, Nitrobenzyl- oder Benzhydrylester.

Als N-acylierte, einen Phenylkern enthaltende Aminocarbonsäuren sind bevorzugt N-Acyl-Derivate von R- oder S-Phenylalanin, –C-Phenylglycin und -Tyrosin.

Als N-Acyl-Schutzgruppen sind die z.B. in Houben-Weyl Bd. XV/1, Seite 46–305 oder in Bodanszky et al., «Peptide Synthesis», 2. Auflage (1976), John Wiley & Sons beschriebenen üblichen $NH_2$-Schutzgruppen brauchbar. Bevorzugt sind Alkanoyl mit 1 bis 6 C-Atomen, insbesondere Formyl, tert.-Butoxycarbonyl und Benzyloxycarbonyl. Eventuell vorhandene freie OH-Gruppen können gegebenenfalls durch Alkyl mit 1 bis 6 C-Atomen, insbesondere Methyl, Ethyl oder tert.-Butyl, durch Benzyl oder andere in der Peptidchemie übliche OH-Schutzgruppen O-alkyliert sein (vgl. z.B. Houben-Weyl, Bd. XV/1 oder Bodanszky et al., «Peptide Synthesis», 2. Auflage (1976), John Wiley & Sons).

Als Lösungsmittel eignen sich bevorzugt aprotische organische Lösungsmittel wie z.B. Ester, Essigester, Cyclohexan, Tetrahydrofuran, doch können auch Alkohole mit bis zu 6 C-Atomen verwendet werden.

Octahydroindol-2-carbonsäure ist aus dem US-Patent 4 350 704 bekannt. 2-Azabicyclo[3.3.0]-octan-3-carbonsäure ist Gegenstand der deutschen Patentanmeldung DE-A-3 226 768 und 2,3,3a,4,5,7a-Hexahydro[1H]indol-2-carbonsäure ist Gegenstand der deutschen Patentanmeldung DE-A-3 210 496. Octahydroisoindol-1-carbonsäure sowie 3-Azabicyclo[3.3.0]-octan-4-carbonsäure sind Gegenstand der deutschen Patentanmeldung DE-A-3 211 676.

Racemische bicyclische cis, endo-Imino-α-carbonsäuren der Formeln Ia + Ib, in welchen C und $B^2$ Wasserstoff und A und $B^1$ gemeinsam die genannte Kette bedeuten, lassen sich beispielsweise aus Enaminen eines Cycloalkanons und N-acylierten β-Halogen-α-amino-carbonsäureestern der Formel IV, in welcher X′ für eine nucleofuge Gruppe, vorzugsweise Chlor oder Brom, Y′ für Alkanoyl mit 1 bis 5 C-Atomen, Aroyl mit 7 bis 9 C-Atomen oder andere, in der Peptidchemie übliche, sauer abspaltbare Schutzgruppen und $R^4$ für Alkyl mit 1 bis 5 C-Atomen oder Aralkyl mit 7 bis 9 C-Atomen steht

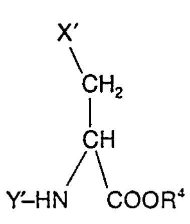

(IV)

oder mit Acrylsäureestern der Formel V, in welcher Y′ und $R^4$ vorstehende Bedeutung haben, herstellen,

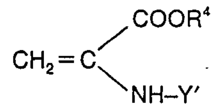

(V)

indem man diese zu Verbindungen der Formel VI, in welcher A, $B^1$, $R^4$ und Y′ vorstehende Bedeutung haben, umgesetzt

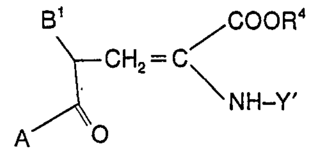

(VI)

diese mit Hilfe starker Säuren unter Acrylamid- und Esterspaltung zu Verbindungen der Formel VIIa oder b cyclisiert,

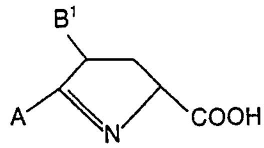

(VIIa)

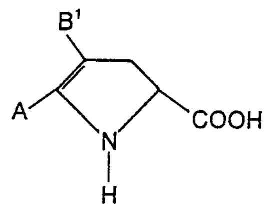

(VIIb)

diese durch katalytische Hydrierung in Gegenwart von übergangsmetallkatalysatoren oder durch Reduktion mit Boran-Aminkomplexen oder komplexen Borhydriden in niederen Alkoholen in Verbindungen der Formeln Ia und Ib, in welchen R für Wasserstoff steht, überführt und diese zu Verbindungen der Formeln Ia und Ib, in welchen R die vorstehend definierte Bedeutung hat, verestert.

Racemische bicyclische Imino-α-carbonsäuren der Formeln Ia und Ib, in welchen A und $B^1$ Wasserstoff bedeuten und $B^2$ und C gemeinsam die genannte Kette bedeuten, lassen sich beispielsweise aus Verbindungen der Formel VIII

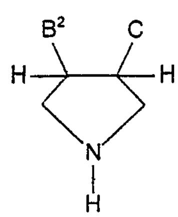

(VIII)

in welcher die Brückenkopf H-Atome zueinander cis- oder trans-orientiert sind und $B^2$ und C vorstehende Bedeutung haben, herstellen.

Verbindungen der Formel VIII mit n = 1 sind aus R. Griot, Helv. Chim. Acta 42, 67 (1959), solche mit n = 2 aus C.M. Rice et al., J. Org. Chem. 21, 1687 (1955) bekannt.

Diese Verbindungen der Formel VIII werden in bekannter Weise acyliert, wobei ein aliphatischer

oder aromatischer Acyl-Rest, vorzugsweise ein Acetyl- oder Benzoylrest an das Stickstoffatom gebunden wird, und die erhaltenen N-acylierten Verbindungen in einem aliphatischen Alkohol, bevorzugt einem Alkohol mit 1 bis 4 C-Atomen, insbesondere Methanol, in Gegenwart eines Leitsalzes vorzugsweise bei Temperaturen im Bereich von 0° bis +40 °C unter Bildung einer Verbindung der Formel IX, worin $B^2$ und C vorstehende Bedeutung haben und

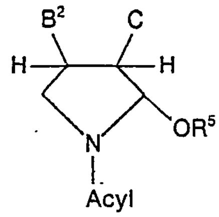

(IX)

$R^5 = (C_1–C_4)$-Alkyl bedeutet, anodisch oxidiert (analog: Liebigs Ann. Chem. 1978, S. 1719).

Die erhaltene Verbindung der allgemeinen Formel IX wird mit Trimethylsilylcyanid nach Tetrahedron Letters 1981, S. 141 in einem Kohlenwasserstoff, Halogenkohlenwasserstoff, in Ether oder in THF bei Temperaturen im Bereich von −60 °C bis +20 °C, vorzugsweise −40 °C bis ± 0 °C in Gegenwart einer Lewis-Säure wie beispielsweise $ZnCl_2$, $SnCl_2$, $SnCl_4$, $TiCl_4$, $BF_3$-Etherat, vorzugsweise $BF_3$-Etherat, umgesetzt und die erhaltene Verbindung der Formel X

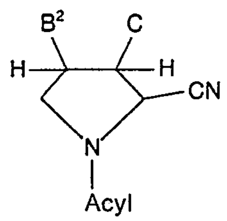

(X)

worin die Brückenkopf H-Atome zueinander cis- oder trans-ständig sind, wobei die CN-Gruppe cisständig zum Brückenkopf-H-Atom an C-Atom (4+n) befindet und worin n, $B^2$ und C die obengenannte Bedeutung haben, nach Reinigung und Auftrennung des Diastereomerengemisches mittels Umkristallisation oder Säulenchromatographie durch Einwirkung von Säuren oder Basen in bekannter Weise zu einer Verbindung der Formeln Ia und Ib mit R = Wasserstoff hydrolysiert und letztere verestert. Bei der sauren Hydrolyse der Nitrilgruppe wird insbesondere HCl oder HBr als Säure verwendet. Die Veresterung wird hier wie im folgenden gemäss den in der Aminosäurechemie üblichen Verfahrensweisen durchgeführt.

Die Erfindung betrifft weiterhin optisch einheitliche Verbindungen der Formel Ia oder Ib, in welchen die beiden Brückenkopf-Wasserstoffatome cis-konfiguriert sind, die COOR-Gruppe endoständig zum bicyclischen Ringsystem orientiert ist, das zu COOR-Gruppe α-ständige C-Atom R oder S konfiguriert ist,

R für Alkyl mit 1 bis 6 C-Atomen, Cycloalkyl mit 4 bis 8 C-Atomen oder Aralkyl mit 7 bis 13 C-Atomen, das gegebenenfalls durch $NO_2$ substituiert sein kann, steht und A, $B^1$, $B^2$ und C wie oben definiert sind und solche Verbindungen der Formeln Ia oder Ib, in welchen R Wasserstoff bedeutet und

a) A und $B^1$ Wasserstoff bedeuten und $B^2$ und G gemeinsam eine Kette der Formel $-[CH_2]_n-$ mit n = 3, 4, 5 oder 6 oder eine Kette der Formel $-[CH_2]_p-CH=CH-[CH_2]_q-$ mit (p+q) = 1, 2, 3 oder 4 bilden oder

b) C und $B^2$ Wasserstoff bedeuten und A und $B^1$ gemeinsam eine der vorstehend unter a) definierten Ketten mit n = 3, 5 oder 6 bzw. (p+q) = 1, 2, 3 oder 4 bilden, sowie deren Salze.

Die Erfindung betrifft ferner diastereomere Salze aus einem bicyclischen cis, endo-Imino-α-carbonsäureester der Formel Ia oder Ib, in welchen A, $B^1$, $B^2$, C und R die oben als bevorzugt definierten Bedeutungen haben und einer N-acylierten, optisch aktiven R- oder S-Aminocarbonsäure, die einen Phenylkern enthält und die, wie vorstehend definiert, geschützt ist.

Die Erfindung betrifft ferner die Verwendung der optisch reinen Verbindungen der Formel Ia bzw. Ib in einem Verfahren zur Herstellung von optisch reinen Verbindungen der allgemeinen Formeln IIa oder IIb

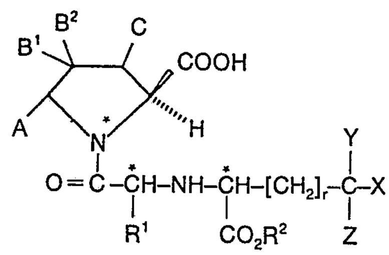

(IIa)

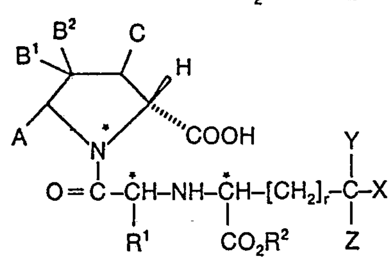

(IIb)

in welchen die mit einem Stern (*) markierten Kohlenstoffatome jeweils unabhängig voneinander die R- oder die S-Konfiguration aufweisen können,

a) A und $B^1$ = Wasserstoff bedeuten und $B^2$ und C gemeinsam eine Kette der Formel $-[CH_2]_n-$ mit n = 3, 4, 5 oder 6 oder eine Kette der Formel $-[CH_2]_p-CH=CH-[CH_2]_q-$ mit (p+q) = 1, 2, 3 oder 4 bilden,

b) C und $B^2$ = Wasserstoff bedeuten und A und $B^1$ gemeinsam eine Kette der Formel $-[CH_2]_n-$ mit n = 3, 4, 5 oder 6 oder eine Kette der Formel $-[CH_2]_p-CH=CH-[CH_2]_q-$ mit (p+q) = 1, 2, 3 oder 4 bilden oder

c) A und C = Wasserstoff bedeuten und $B^1$ und $B^2$ gemeinsam eine Kette der Formel $-[CH_2]_m-$ mit m = 4, 5, 6 oder 7 bilden,

r = 0 oder 1,

$R^1$ = Wasserstoff, einen gegebenenfalls substituierten aliphatischen Rest mit 1 bis 6 C-Atomen,

einen gegebenenfalls substituierten alicyclischen Rest mit 3 bis 9 C-Atomen, einen gegebenenfalls substituierten alicyclisch-aliphatischen Rest mit 4 bis 11 C-Atomen, einen gegebenenfalls substituierten aromatischen Rest mit 6 bis 12 C-Atomen, der auch teilhydriert sein kann, einen gegebenenfalls substituierten araliphatischen Rest mit 7 bis 15 C-Atomen, einen gegebenenfalls substituierten aroylaliphatischen Rest mit 8 bis 13 C-Atomen, einen gegebenenfalls substituierten mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer natürlich vorkommenden gegebenenfalls geschützten Aminosäure,

$R^2$ = Wasserstoff, einen gegebenenfalls substituierten aliphatischen Rest mit 1 bis 6 C-Atomen, einen gegebenenfalls substituierten araliphatischen Rest mit 7 bis 15 C-Atomen,

Y = Wasserstoff oder Hydroxy,

Z = Wasserstoff oder

Y und Z = zusammen Sauerstoff und

X = einen aliphatischen Rest mit 1 bis 6 C-Atomen, einen alicyclischen Rest mit 5 bis 9 C-Atomen, einen gegebenenfalls substituierten aromatischen Rest mit 6 bis 12 C-Atomen oder Indolyl bedeuten, das

dadurch gekennzeichnet ist, dass man optisch reine Verbindungen der Formeln Ia oder Ib, in welchen A, $B^1$, $B^2$ und C die obengenannte Bedeutung besitzen und

R für einen gegebenenfalls substituierten aliphatischen Rest mit 1 bis 6 C-Atomen, einen gegebenenfalls substituierten alicyclischen Rest mit 4 bis 10 C-Atomen, einen gegebenenfalls substituierten aromatischen Rest mit 6 bis 12 C-Atomen oder einen gegebenenfalls substituierten araliphatischen Rest mit 7 bis 15 C-Atomen steht, mit optisch reinen Verbindungen der Formel III

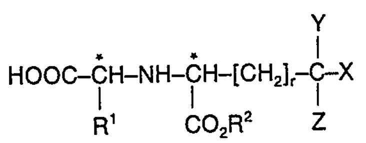

(III)

in der die beiden mit einem Stern (*) markierten C-Atome die Konfiguration (S, R), (R, S), (R, R) oder vorzugsweise (S, S) aufweisen und

r, $R^1$, $R^2$, X, Y und Z die obengenannte Bedeutung besitzen, in Anwesenheit eines Kondensationsmittels oder gegebenenfalls als Aktivester umsetzt, den Rest R durch Hydrogenolyse oder Hydrolyse abspaltet und die optisch reinen Verbindungen der Formeln IIa oder IIb gegebenenfalls in physiologisch verträgliche Salze überführt.

Eine bevorzugte Ausführungsform des erfindungsgemässen Verfahrens ist dadurch gekennzeichnet, dass Verbindungen der Formeln IIa oder IIb hergestellt werden, in denen

r = 0 oder 1,

R = Wasserstoff, ($C_1$ bis $C_6$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$)-Cycloalkyl-($C_1$ bis $C_4$)-alkyl, Aryl oder teilhydriertes Aryl mit 6 bis 12 C-Atomen, das jeweils durch ($C_1$ bis $C_4$)-Alkyl, ($C_1$ oder $C_2$)-Alkoxy oder Halogen substituiert sein kann, ($C_6$ bis $C_{12}$)-Aryl-($C_1$–$C_4$)-alkyl oder ($C_7$–$C_{13}$)-Aroyl-($C_1$–$C_2$)alkyl die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden Aminosäure,

$R^2$ = Wasserstoff, ($C_1$ bis $C_6$)-Alkyl, ($C_2$ bis $C_6$)-Alkenyl oder ($C_6$ bis $_{12}$)-Aryl-($C_1$ bis $C_4$)-alkyl,

Y = Wasserstoff oder Hydroxy,

Z = Wasserstoff oder

Y und Z = zusammen Sauerstoff und

X = ($C_1$ bis $C_6$)-Alkyl, ($C_2$ bis $C_6$)-alkenyl, ($C_5$ bis $C_9$)-Cycloalkyl, ($C_6$ bis $C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)-alkylamino und/oder Methylendioxy mono-, di- oder trisubstituiert sein kann, oder 3-Indolyl bedeuten.

Bevorzugt ist die Herstellung der S,S,S-Verbindungen der Formel IIa.

Unter Aryl ist hier wie im folgenden vorzugsweise gegebenenfalls substituiertes Phenyl oder Naphthyl zu verstehen. Alkyl kann geradkettig oder verzweigt sein.

Unter einem mono- bzw. bicyclischer Heterocyclen-Rest mit 5 bis 7 bzw. 8 bis 10 Ringatomen, in welchem die Ringatome die oben genannten Bedeutungen haben, wird beispielsweise Thienyl, Benzo[b]-thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Falls $R^1$ für eine Seitenkette einer geschützten natürlich vorkommenden α-Aminosäure steht, wie z. B. geschütztes oder gegebenenfalls substituiertes Ser, Thr, Asp, Asn, Glu, Gln, Arg, Lys, Hyl, Cys, Orn, Cit, Tyr, Trp, His oder Hyp, sind als Schutzgruppen die in der Peptidchemie üblichen Gruppen bevorzugt (vgl. Houben-Weyl, Bd. XV/1 und XV/2). Im Falle, dass $R^1$ die geschützte Lysin-Seitenkette bedeutet, werden die bekannten Amino-Schutzgruppen, insbesondere aber ($C_1$–$C_6$)-Alkanoyl bevorzugt. Bevorzugte O-Schutzgruppen für Tyrosin sind Methyl oder Ethyl.

Bei den bisher bekannten, von Stereoisomerengemischen ausgehenden Verfahrensweisen, die zur Herstellung von Stereoisomerengemischen von Verbindungen der Formeln IIa oder IIb dienten, war es notwendig, das Reaktionsgemisch in aufwendigen Trennprozeduren aufzutrennen, um

das gewünschte optisch reine Stereoisomere der Formel IIa oder IIb zu erhalten.

Durch Umsatz der erfindungsgemässen, optisch einheitlichen Imino-α-carbonsäureester der Formeln Ia oder Ib mit optisch einheitlichen Verbindungen der Formel III ist die gezielte Synthese optisch einheitlicher Verbindungen der Formel IIa oder IIb möglich geworden. Die gewünschten Verbindungen der Formeln IIa oder IIb werden in hohen Ausbeuten ohne Anwendung aufwendiger Trenntechniken erhalten.

Verbindungen der Formel III sind in den vorstehend genannten Dokumenten beschrieben oder sind aus der EP-A-46 953 bekannt. Der Umsatz einer Verbindung der Formel III mit 1-(2α,3aβ,-7aβ)-Octahydro-[1H]-indol-2-carbonsäure tert.-butylester und die anschliessende Abspaltung eines tert.-Butylesters, wobei ein entsprechendes Octahydroindol-Derivat der Formel IIb resultiert, ist aus der EP-A-37 231 bekannt.

Dieser Umsatz musste sich jedoch auf die Reaktion einer Verbindung der Formel Ia bzw. Ib mit C, $B^2$ jeweils = H und A + $B^1$ = $(CH_2)_4$ beschränken, die bisher nur auf umständliche Weise über die N-Benzoylverbindung, Kristallisation der diastereomeren Salze mit S-α-Phenylethylamin, Freisetzung der N-Benzoylverbindung, Abspaltung der Benzoylgruppe und Veresterung hergestellt werden konnte.

Die Übertragung dieser Reaktionsfolge auf die erfindungsgemässen Zwischenprodukte der Formel Ia und Ib ist bisher nicht gelungen. Auch die Trennung racemischer Gemische von Verbindungen der Formel Ia und Ib durch Trennung gängiger diastereoisomerer Salze mit optisch aktiven Carbon- oder Sulfonsäuren gelang nicht. Erst durch die oben beschriebene Arbeitsweise wurden die Verbindungen der Formel Ia und Ib für Folgereaktionen zugänglich.

Das erfindungsgemässe Verfahren ist besonders ökonomisch, da Verbindungen der Formel III nach der Deutschen Patentanmeldung P 3 226 768.1 auf einfachem Wege direkt in optisch reiner Form hergestellt werden können. Dort mussten diese Zwischenprodukte allerdings noch mit einer racemischen Aminosäure umgesetzt und durch einen zusätzlichen Reinigungsschritt in eine optisch reine Verbindung der allgemeinen Formeln IIa bzw. IIb übergeführt werden.

Das erfindungsgemässe Verfahren, das bevorzugt mit S,S-Verbindungen der Formel III durchgeführt wird, stellt somit für die Herstellung der umfassten Verbindungen das bei weitem ökonomischste Verfahren dar, da bei allen anderen bekannten Verfahrensweisen hohe Verluste durch Chromatographie oder Kristallisation teilweise komplexer Stereoisomerengemische hingenommen werden mussten.

Der Kondensationsschritt erfolgt nach einer der gängigen, racemisierungsarm verlaufenden Verfahren der Peptidsynthese, wie sie z.B. in Houben-Weyl, Band XV, oder in «The Peptides, Analysis, Synthesis, Biology, Vol. 1 Major Methods of Peptide Bond Formation, Part A», Gross, Meierhofer, Academic Press N.Y. (1979) beschrieben sind.

Besonders vorteilhaft ist die DCC/HOBt-Methode, nach Chem. Ber. 103 (1979), Seiten 788–798. Hierbei ist zu berücksichtigen, dass reaktionsfähige funktionelle Gruppen im Rest $R^1$ nach den bekannten Methoden der Peptidchemie (z.B. Houben-Weyl, Band XV oder Bodanszky et al. in «Peptide Synthesis», 2. Auflage (1976), John Wiley & Sons) vorübergehend geschützt werden müssen.

Die optisch einheitlichen Verbindungen der Formel IIa bzw. IIb werden in an sich bekannter Weise nach Abspalten von R und gegebenenfalls $R^2$ in hoher Ausbeute ohne Anwendung aufwendiger Trenntechniken erhalten.

Die Verbindungen der Formel IIa bzw. IIb und deren Salze besitzen lang andauernde, intensive blutdrucksenkende Wirkung. Sie sind starke Hemmer des Angiotensin-Converting-Enzyms (ACE) und können zur Bekämpfung des Bluthochdrucks verschiedener Genese eingesetzt werden. ACE-Inhibitoren dieses Typs sind z.B. aus dem US-Patent 4 344 949, der EP-A-49 658, der EP-A-46 953, der EP-A-50 800 und der EP-A-79 022 bekannt.

Auch ihre Kombination mit anderen blutdrucksenkenden, gefässerweiternden oder diuretisch wirksamen Verbindungen ist möglich. Typische Vertreter dieser Wirkklassen sind z.B. in Erhardt-Ruschig, Arzneimittel, 2. Auflage, Weinhein, 1972, beschrieben. Die Anwendung kann intravenös, subcutan oder peroral erfolgen.

Die Dosierung bei oraler Gabe liegt gewöhnlich bei 1–500 mg vorzugsweise bei 1–100 mg je Einzeldosis bei einem normalgewichtigen erwachsenen Patienten. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemässen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch üblichen Methoden in geeignete Darreichungsformen gebracht, wie Tabletten, Dragees, Steckkapseln, wässrige, alkoholische oder ölige Suspensionen oder wässrige, alkoholische oder ölige Lösungen. Als inerte Träger können z.B. Gummi arabicum, Magnesiumcarbonat, Kaliumphosphat, Milchzukker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche oder tierische Öle in Betracht, wie Sonnenblumenöl oder Lebertran.

Zur subkutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weiteren Hilfsstoffen in Lösung, Suspension oder Emulsion gebracht.

Als Lösungsmittel für die neuen aktiven Verbindungen und die entsprechenden physiologisch verträglichen Salze kommen z.B. in Frage: Wasser, physiologische Kochsalzlösung oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln oder Lösungen.

Die nachfolgenden Beispiele erläutern das Verfahren, ohne dass die Erfindung auf diese speziellen Beispiele beschränkt wäre.

Beispiel 1:
(1S, 3S, 5S)-2-Azabicyclo[3.3.0]octan-3-carbonsäurebenzylester-hydrochlorid (Abk. (S)-Aoc-OBzl-HCl)
(A) 2-Acetylamino-3-(2-oxo-cyclopentyl)-propionsäuremethylester:

269 g 3-Chlor-2-acetyl-amino-propionsäuremethylester und 257 g Cyclopentenopyrrolidin werden in 1,5 l DMF 24 Stunden bei Raumtemperatur gehalten. Man engt im Vakuum ein, nimmt den Rückstand in wenig Wasser auf, stellt mit konzentrierter Salzsäure auf pH 2 und extrahiert 2 mal mit je 4 l Essigester. Beim Einengen der organischen Phase hinterbleibt ein hellgelbes Öl.

Ausbeute: 290 g.

$^1$H-NMR: 2,02 (s, 3H); 3,74 (s, 3H); 4,4–4,8 (m, 1H), ($CDCl_3$)

Analyse:

| | | | |
|---|---|---|---|
| ber. | C 58,1 | H 7,54 | N 6,16 |
| gef. | C 58,5 | H 7,2 | N 6,5 |

(B) cis,endo-2-Azabicyclo-[3.3.0]-octan-3-carbonsäurehydrochlorid

270 g des unter (A) hergestellten Acetylamino-Derivates werden in 1,5 l 2n Salzsäure 45 Minuten am Rückfluss gekocht. Man engt im Vakuum ein, nimmt den Rückstand in Eisessig auf, versetzt mit 5 g Pt/C (10% Pt) und hydriert bei 5 bar. Nach Filtration wird eingeengt und der Rückstand aus Chloroform/Diisopropylether kristallisiert.

Schmelzpunkt: 205–209 °C,
Ausbeute: 150 g

(C) Racemisches Aoc-OBzl · HCl

1,2 l (11,5 Mol) Benzylalkohol werden auf −10 °C gekühlt. Man tropft unter Kühlen und Rühren 126 ml (1,73 Mol) Thionylchlorid zu und trägt anschliessend bei −10 °C unter Rühren 126,5 g (0,66 Mol) rohes Aoc.HCl ein und rührt 30 Minuten bei dieser Temperatur nach. Dann lässt man die Temperatur langsam auf 20–25 °C ansteigen, wobei sich das Produkt unter Rühren innerhalb von 5 stunden löst. Nach Stehen über Nacht lässt man die braune Lösung unter Rühren in 4,0 l Diisopropylether einlaufen. Nach 1 Stunde filtriert man die ausgefallenen Kristalle ab, wäscht sie mit Diisopropylether und trocknet im Vakuum. Aus den vereinigten Diisopropyletherlösungen fällt über Nacht ein weiterer Niederschlag aus.

Ausbeute: 168,5 g (90.6%)

(D) (S)-Aoc-OBzl · Z–Phe–OH

166,0 g (0,589 Mol) racemisches Aoc-OBzl.HCl werden in 500 ml Methylenchlorid suspendiert und mit 25 g (0,625 Mol) NaOH in 250 ml Wasser gut geschüttelt. Es tritt Lösung ein. Nach kurzer Zeit hat sich die zunächst gebildete Emulsion getrennt. Man trennt die Methylenchloridphase ab, wäscht mit 100 ml 0,1 n NaOH und zweimal mit je 50 ml Wasser und extrahiert die vereinigten wässrigen Phasen zweimal mit je 100 ml Methylenchlorid. Die zusammengefassten Methylenchloridphasen werden über Natriumsulfat getrocknet und im Wasserstrahlvakuum schonend eingeengt. Das zurückbleibende Öl wird sogleich in 100 ml Essigester aufgenommen und mit der Lösung von 117,6 g (0,39 Mol) N-Benzyloxycarbonyl-S-phenylalanin (Z-Phe-OH) in 200 ml Essigester versetzt. Der Kolben wird mit 100 ml Essigester nachgewaschen. Die klare Lösung wird unter Rühren bei Raumtemperatur mit 1600 ml Cyclohexan (= 4-fache Vol-Menge) versetzt. Nach Anreiben beginnt die Kristallisation, die durch Stehen im Kühlraum über Nacht vervollständigt wird. Man filtriert den kristallinen Niederschlag ab, wäscht mit 250 ml Essigester/Cyclohexan (1 + 4) und trocknet.

Ausbeute: 133,6 g (S)-Aoc-OBzl · Z-Phe-OH (50,9%, entspr. 102% d.Th.),

Schmp. 101–103 °C: $[\alpha]_D^{27}$: −5,3° (c = 1 Methanol)

Nach Umkristallisieren aus Essigester/Cyclohexan (1:1) findet man für ds Z-Phe-OH-Salz folgende Daten:

Schmp: 103–104 °C, $[\alpha]_D^{27}$: −6,1° (c = 1, in Methanol).

(E) (S)-Aoc-OBzl · HCl

63,0 g (0,142 Mol) des nach (D) erhaltenen Z-Phe-OH-Salzes werden in 300 ml Methylenchlorid gelöst, mit 6,0 g (0,15 Mol) NaOH in ca. 150 ml Wasser gut geschüttelt. Die Phasentrennung nimmt wegen wenig ungelösten Anteilen etwas Zeit in Anspruch. Man trennt die Methylenchloridphase ab, wäscht sie mit 50 ml 0,1 n NaOH, zweimal mit je 50 ml Wasser und trocknet. Man engt auf ca. 100 ml ein, verdünnt mit 100 ml Diisopropylether und versetzt unter Rühren mit 25 ml 6 n HCl in Ether. Nach 1 Stunde wird filtriert, der Niederschlag mit Diisopropylether gewaschen und getrocknet.

Ausbeute: 32,5 g (81,3%)
Schmp: 185–186 °C
$[\alpha]_D^{30}$: −42,5° (c = 1, Wasser)

Die basische Wasserphase wird im Vak. von Methylenchlorid befreit und mit konz. HCl angesäuert. Das ausgefallene Z-Phe-OH wird mit Wasser gewaschen und getrocknet.

Auf die beschriebene Weise werden aus der Methylenchlorid-Mutterlauge von Beispiel I (D) die R-Verbindung und weiteres Z-Phe-OH erhalten.

Tabelle 1

| Beispiel | Iminocarbonsäure | Ester R | Säure | Lösungsmittel | Diastereoisomere Salze Schmp. | $[\alpha]_D^{20}$ (c = 1, MeOH) | Säurekomponente | Endprodukt S-Form Schmp. | $[\alpha]_D^{20}$ (c = 1, $H_2O$) | R-Form Schmp. | $[\alpha]_D^{20}$ (c = 1, $H_2O$) |
|---|---|---|---|---|---|---|---|---|---|---|---|
| 2 | XIII | Methyl | Z-Phe-OH | Diethylether | 115–116° C | + 5,8° | TosOH | 191–192° C | −12,9° | 190–192° C | +12,6° |
| 3 | XIII | Benzyl | Z-Phe-OH | Essigester/Cyclohexan | 104–105° C | + 6,2° | HCl | 184–186° C | −41,2° | 183–185° C | +42,5° |
| 4 | XIII | Benzyl | Z-Phe-OH | Isopropanol | 103–104° C | − 6,0° | HCl | 182–185° C | −39,9° | | |
| 5 | XIII | Benzyl | Z-R-Phe-OH | Essigester/Cyclohexan | 102–104° C | + 4,4° | HCl | | | 185–186° C | +41,9° |
| 6 | XIII | Benzyl | Z-Pgl-OH | Essigester | 128–130° C | +32,1° | HCl | 184–185° C | −40,9° | | |
| 7 | XIII | Benzyl | Z-Tyr-OH | Essigester/Cyclohexan | 125–126° C | − 0,4° | HCl | 184–185° C | −41,6° | | |
| 8 | XIII | Benzyl | Z-Tyr (But)-OH | Essigester/Cyclohexan | 104–105° C | − 3,4° | HCl | 181–183° C | −40,0° | | |
| 9 | XIII | Benzyl | For-Phe-OH | Isopropanol | 107–109° C | + 1,7° | HCl | 178–181° C | −39,1° | | |
| 10 | XIII | Nitrobenzyl | Z-Phe-OH | Essigester | 122–124° C | − 1,3° | TosOH | | −29,8° | | |
| 11 | XI | Benzyl | Z-Phe-OH | Essigester/Cyclohexan | 106–107° C | −13,5° | TosOH | 152 − 153° C | −36,2° | | |
| 12 | XII | Methyl | Z-Phe-OH | Essigester/Cyclohexan | 107–108° C | +47,1° | HCl | | +68,4° | | |

In analoger Weise werden die [cis-endo]-Imino-α-carbonsäureester von Tabelle 1 hergestellt und einer Racematspaltung unterworfen. In dieser Tabelle sind ihre optisch aktiven Kristallisationspartner, Lösungsmittel, Ausbeuten und Eigenschaften der Salze und der Endprodukte in Form der Esterhydrochloride oder -tosylate aufgeführt.

Erläuterungen zu Tabelle 1

(XI) 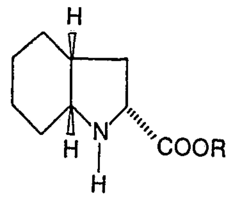 und Spiegelbild

(XII) 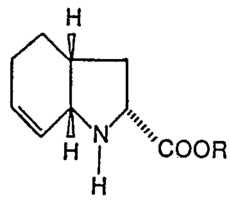 und Spiegelbild

(XIII) 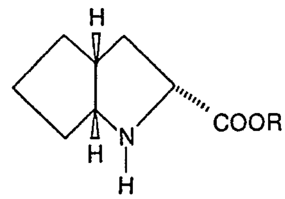 und Spiegelbild

Aus den Estern können die freien Iminocarbonsäuren durch Verseifung bzw. Hydrogenolyse hergestellt werden.

Beispiel 13

N-(1S-Carbethoxy-3-phenyl-propyl)-S-alanyl-2-cis,endo-azabicyclo[3.3.0]octan-3S-carbonsäure

(A) N-(2S-Carbethoxy-3-phenyl-propyl)-S-alanyl-cis-endo 2-azabicyclo[3.3.0]octan-3S-carbonsäurebenzylester

14 g des nach Beispiel 1 E hergestellten Benzylesterhydrochlorids werden durch Ausschütteln der alkalischen wässrigen Lösung mit Diethylether in den freien Ester übergeführt, der nach Abdestillieren des Ethers mit 6,7 g HOBt, 13,8 g N-(1S-Carbethoxy-3-phenylpropyl)-S-alanin, und 10,2 g Dicyclohexylcarbodiimid in 200 ml Dimethylformamid zur Reaktion gebracht wird. Nach 3 Stunden Rühren bei Raumtemperatur saugt man von ausgefallenem Dicyclohexylharnstoff ab, engt ein, nimmt in 1 l Essigester auf und schüttelt mit 3 × 500 ml 5-prozentiger $NaHCO_3$-Lösung aus. Die organische Phase wird eingeengt.

Es werden 22,4 g (90%) Produkt aus Öl erhalten.

$^1$H-NMR der S,S,S-Verbindung, charakteristische Signale: 1.20 (d, 3H), 1,27 (t, 2H), 4,17 (q, 3H), 5,13 (s, 2H), 7,18 (s, 5H), 7,32 (s, 5H), ($CDCl^3$)

Analyse: $C_{30}H_{38}N_2O_5$

ber. C 71,1 H 7,56 N 5,53

gef. C 70,8 H 7,8 N 5,7

(B) N-(1S-Carbethoxy-3-phenyl-propyl)-S-alanyl-cis,endo-2-azabicyclo[3.3.0]octan-3-S-carbonsäure

8,0 g des S,S,S-Benzylesters aus Beispeil 1E werden in 100 ml Ethanol gelöst und unter Zusatz von 0,5 g 10% Pd/C bei Normaldruck hydrogenolytisch entbenzyliert. Diese Reaktion kann auch unter Druck bei gleichzeitiger Verkürzung der Reaktionszeit vorgenommen werden. Nach Aufnahme der berechneten Menge Wasserstoff wird vom Katalysator abfiltriert und im Vakuum eingeengt. Das Zwitterion kristallisiert in fast quantitativer Ausbeute aus Ether:

Schmelzpunkt: 110–112 °C (Zers.)

Durch Zusatz einer äquivalenten Menge Salzsäure kann ein Hydrochlorid (ab 120 °C Zersetzung) erhalten werden.

Analyse: $C_{23}H_{32}N_2O_5$

ber. C 66,3 H 7,7 N 6,73

gef. C 66,1 H 7,8 N 6,6

Die erhaltenen $^1$H-NMR- und die Massenspektren sind im Einklang mit der angegebenen Struktur.

$[\alpha]_D = +15,6°$ (c = 1, Methanol).

Beispiel 14

N-(1 S-Carbethoxy-2-benzoyl-ethyl)-O-ethyl-S-tyrosyl-cis,endo-2-azabicyclo[3.3.0]-octan-3 S-carbonsäure

(A) N-(1 S-Carbethoxy-2-benzoyl-ethyl)-O-ethyl-S-tyrosin-benzylester

Man setzt 24 g Benzoylacrylsäure-ethylester in 100 ml Ethanol mit 30 g O-Ethyl-S-tyrosin-benzylester in Anwesenheit von 0,5 ml Triethylamin um und erhält nach Einengen der Lösung und Digerieren des Rückstandes mit Diethylether/Petrolether (1:1) und Trocknen im Vakuum 42 g RS,S-Verbindung. Diastereomerentrennung durch Chromatographie an Kieselgel im System Essigester/Cyclohexan (1:3).

Ausbeute: 17 g der S,S-Verbindung.

(B) N-(1S-Carbethoxy-2-benzoyl-ethyl)-O-ethyl-S-tyrosin

17 g der nach (A) erhaltenen Verbindung werden in 800 ml Essigsäure mit 4 g Pd/C (10%) bei 100 bar und Raumtemperatur hydriert. Ausbeute nach Chromatographie an Kieselgel im Lösungsmittel Essigester/Cyclohexan (1:3) und Trocknen des Eindampfrückstandes: 12 g dünnschicht-chromatographisch nahezu einheitliche Titelverbindung.

Schmp. 205–213 °C

Analyse: $C_{23}H_{29}NO_5$ (399,5)

Ber. C 69,15 H 7,31 N 3,50

Gef. C 69,5 H 7,4 N 3,3

(C) N-(1S-Carbethoxy-2-benzoyl-ethyl)-O-ethyl-S-tyrosyl-cis,endo-2-azabicyclo[3.3.0]octan-3S-carbonsäure

Man setzt analog Beispiel 13 A 8 g der nach Beispiel 1 E und Ausschütteln mit Diethylether aus alkalischer Lösung erhaltenen freien Benzylesters mit 8 g der nach Beispiel 14 B erhaltenen Verbindung mittels 4,4 g Dicyclohexylcarbodiimid in Anwesenheit von 2,7 g 1-Hydroxybenzotriazol um und erhält 14,3 g öligen Benzylester als Zwischenprodukt.

Die $^1$H-NMR- und die Massenspektren sind im Einklang mit der angegebenen Struktur.

Der Benzylester wird in 50 ml Ethanol unter Normaldruck an PdlC katalytisch hydriert. Nach Abfiltrieren des Katalysators und Abdestillieren des Lösungsmittels bleibt ein fester Rückstand zurück, der mit Diethylether/Petrolether digeriert und getrocknet wird.

Ausbeute: 11,2 g.

Beispiel 15

N-(1 S-Carbethoxy-3-phenyl-propyl)-O-methyl-S-tyrosyl-cis-endo-2-azabicyclo[3.3.0] octan-3 S-carbonsäure

Man arbeitet wie in Beispiel 14 beschrieben, setzt aber in der (A) analogen Stufe O-Methyl-S-tyrosin-benzylester ein und erhält die Titelverbindung, deren $^1$H-NMR-Spektrum im Einklang mit der angegebenen Struktur ist.

$^1$H-NMR ($CDCl_3$): 1,2–3,0 (m, 15H); 1,27 (t, 3H); 1,4 (t, 3H); 3,0–4,3 (m, 4H); 3,8–4,2 (m, 4H); 6,5–7,1 (2d, 4H); 7,3 (s, 5H)

Beispiel 16

N-(1 S-Carbethoxy-3-phenyl-propyl)-S-alanyl-2-aza-spiro[4.5] decan-3 S-carbonsäure

(A) 1-(Diethoxyethyl)-cyclohexancarbonitril

Zu 312,5 ml (0,5 Mol) einer 15%igen Lösung von n-Butyllithium in Hexan werden bei −10 °C unter Schutzgas 51,7 ml (0,5 Mol) wasserfreies Diethylamin getropft. Man rührt 20 Minuten und kühlt dann auf −70 °C ab. Im Verlauf von 30 Minuten werden 54,6 g Cyclohexancarbonitril eingetropft, nach weiteren 30 Minuten innerhalb 1 Stunde 98,5 g Bromacetaldehyd-diethylacetal zugefügt und 24 Stunden bei tiefer Temperatur belassen. Man erwärmt auf Raumtemperatur gibt 100 g Eis hinzu, extrahiert zweimal mit 500 ml Essigester, trocknet die organische Phase über Natriumsulfat, engt im Vakuum ein und unterwirft den Rückstand der Vakuumdestillation.

Ausbeute: 90 g (rund 80% d.Th.), Kp. 78–79 °C bei 8 Torr (10,7 mbar)

(B) 1-Aminomethyl-1-(diethyloxyethyl)-cyclohexan

90 g Diethoxyethyl-cyclohexancarbonitril werden in 1 l Ethanol gelöst und mit 60 g Natrium versetzt. Nach Auflösung des Metalls werden 100 ml Wasser zugefügt und das Lösungsmittel wird weitgehend im Vakuum entfernt. Der Rückstand wird mit 300 ml Wasser versetzt und 3x mit 200 ml Ether extrahiert. Man trocknet die etherische Phase über Natriumsulfat, engt ein und destilliert im Vakuum.

Ausbeute: 83 g (etwa 90% d.Th.), Kp. 69–72 °C bei 8 Torr (10,7 mbar)

(C) 2-Aza-spiro[4.5]decan-3-carbonitril

80,2 g Aminomethyl-diethyloxyethyl-cyclohexan werden unter Schutzgas ($N_2$ oder Ar) in einem Gemisch aus 300 ml Ethanol und 300 ml 1 n Salzsäure ca. 1 Stunde gerührt. Nach vollständiger Spaltung des Ausgangsproduktes wird auf 0 °C gekühlt und die Lösung durch Zugabe von 2 n Natronlauge schnell auf pH 5 gebracht. Man versetzt sofort mit 300 ml Eisessig (pH etwa 3), kühlt auf −10 °C und fügt 17,5 g Natriumcyanid zu. Das Reaktionsgefäss wird verschlossen und etwa 5 Stunden bei Raumtemperatur belassen. Man kontrolliert mit Hilfe der Dünnschichtchromatographie (System Essigester/Petrolether 2:1) auf vollständige Umsetzung (Schiff'sche Base $R_f$ = 0,6–0,7; Aminosäurenitril $R_f$ = 0,28) und engt die Reaktionslösung zur Trockene ein. Das rohe Aminosäurenitril wird alsbald nach Beispiel 16 D oder E weiter verarbeitet.

(D) 2-Aza-spiro[4.5]decan-3-carbonsäure

Die Hälfte des in Beispiel 16 C erhaltenen Aminosäurenitrils wird mit 250 ml 4 n Salzsäure versetzt und 4 Stunden am Rückfluss erhitzt. Spuren von entweichender Blausäure werden auf geeignete Weise (Ausfrieren, Absorption in basischer Eisen-(II)-Salzlösung unschädlich gemacht. Die Lösung wird neutralisiert, zur Trockne gebracht und mehrfach mit n-Butanol extrahiert. Der Abdampfrückstand der organischen Phase wird

a) zur Gewinnung des Hydrochlorids aus Chloroform-Diisopropylether kristallisiert und wenn nötig nochmals aus einem Gemisch mit Ethanol umgefällt oder

b) durch Ausrühren mit Ionenaustauscher, z.B. IR 45 (OH-Form) (Amberlite (R)) in wässriger Lösung gereinigt und das Zwitterion nach Entfernen des Wassers aus Ethanol/Ether kristallisiert.

Ausbeute nach a): 31–32 g (82%)

Schmp. 205 °C (Zers.), Hydrochlorid

(E) 2-Aza-spiro[4.5]decan-3-carbonsäurebenzylester-hydrochlorid

Die Hälfte des nach Beispiel 16C gewonnenen Aminosäurenitrils wird in 70 ml Benzylalkohol aufgenommen. Man leitet bei Raumtemperatur 5 Minuten einen langsamen HCl-Gasstrom durch die Lösung, hält 2–3 Stunden bei Raumtemperatur, engt im Vakuum gut ein, versetzt mit wässriger Natriumbicarbonatlösung bis pH 8,5 erreicht ist und extrahiert den Benzylester in Essigester. Die organische Phase wird getrocknet, mit einer äquivalenten Menge etherischer Salzsäure versetzt und eingeengt. Der Rückstand kristallisiert aus Diisopropylether und kann aus Methylenchlorid/Diisopropylether umkristallisiert werden.

Ausbeute: 43 g (ca. 80%)

Schmp. 145 °C (Zers.)

(F) 2-Aza-spiro[4.5]decan-3S-carbonsäurebenzylesterhydrochlorid

Das nach Beispiel 16 E erhaltene racemische Hydrochlorid wird analog den Beispielen 1 D und E einer Racematspaltung unterworfen.

(G) N-(1 S-Carbethoxy-3-phenyl-propyl)-S-alanyl-2-aza-spiro-[4.5]nonan-3S-carbonsäure-benzylester

15,6 g Spiro[4.5]-2-azanonan-3S-carbonsäurebenzylester-hydrochlorid, 6,7 g 1-Hydroxybenzotriazol und 13,8 g (S,S)-N-(1-Carbethoxy-3-phenylpropyl)-alanin werden in 200 ml DMF gelöst und mit 10,2 g Dicyclohexylcarbodiimid über Nacht zur Reaktion gebracht. Zusatz von tert. Basen, z.B. 6,4 ml N-Ethylmorpholin erhöht die Ausbeute nur unwesentlich. Man filtriert vom ausgefallenen DC-Harnstoff ab, engt das Filtrat im Vakuum ein, nimmt in Essigester auf, schüttelt mit wässriger Natriumbikarbonatlösung aus, trocknet die organische Phase über festem Natriumsulfat und engt erneut ein. Die $^1H$-NMR-Spektren (in $CDCl_3$) bestätigen die Struktur.

(H) N-(1S-Carbethoxy-3-phenyl-propyl)-S-alanyl-2-aza-spiro[4.4]nonan-3S-carbonsäure

Das in Beispiel 16 G erhaltene Benzylester wird in 200 ml Methanol aufgenommen und mit 1 g Pd/C (10% Pd) hydrogenolytisch entbenzyliert. Nach beendeter Wasserstoffaufnahme wird filtriert und im Vakuum eingeengt. Unter Zusatz von Pentan kann im Vakuum ein fester, hygroskopischer Schaum des zwitterionischen Dipeptidderivates erhalten werden. $[\alpha]_D^{21}$ = 38,3 (c = 1, Methanol)

Beispiel 17

N-(1 S-Carbethoxy-3-phenyl-propyl)-S-alanyl-cis,-endo-2,3,3a,4,5,7a-hexahydro[1H]indol-2S-carbonsäure

(A) cis-2,3,3a,4,5,7a-Hexahydro [1H]indol-2-S-carbonsäuremethylester-hydrochlorid

Racemisches cis-2,3,3a,4,5,7a-Hexahydro[1H]-indol-2-carbonsäuremethylester-hydrochlorid (erhältlich analog der in der deutschen Patentanmeldung P 3 210 496.0 beschriebenen Verfahrensweise) wird analog den Beispielen 1 D und E einer Racematspaltung unterworfen.

$[\alpha]_D$ = +68,4° (c=1, $H_2O$)

(B) N-(1S-Carbethoxy-3-phenyl-propyl)-2-alanyl-cis,endo-2,3,3a,4,5,7a-hexahydro[1H]indol-2 S-carbonsäure-hydrochlorid

Man erhält die Titelverbindung analog der in den Beispielen 13 A und B beschriebenen Verfahrensweise.

$^1H$-NMR-Daten 0,9–3,0 (m, 17 H); 3,4–4,9 (m, 6 H); 5,2–6,0 (m, 2 H); 7,2 (s, 5 H)

**Patentansprüche für die Vertragsstaaten BE CH DE FR GB IT LI LU NL SE**

1. Verfahren zur Auftrennung racemischer Gemische bicyclischer Imino-α-carbonsäureester in die Komponenten der Formel Ia und Ib

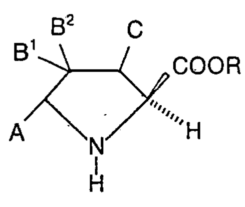

(Ia)

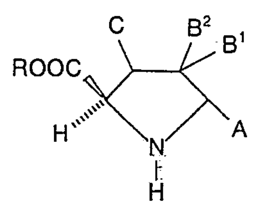

(Ib)

in welchen

R für einen aliphatischen Rest mit 1 bis 6 C-Atomen, einen alicyclischen Rest mit 4 bis 10 C-Atomen, einen aromatischen Rest mit 6 bis 12 C-Atomen oder einen araliphatischen Rest mit 7 bis 15 C-Atomen steht,

a) A und $B^1$ = Wasserstoff bedeuten und $B^2$ und C gemeinsam eine Kette der Formel $-[CH_2]_n-$ mit n = 3, 4, 5 oder 6 oder eine Kette der Formel $-[CH_2]_p-CH=CH-[CH_2]_q-$ mit (p+q) = 1, 2, 3 oder 4 bilden,

b) C und $B^2$ = Wasserstoff bedeuten und A und $B^1$ gemeinsam eine Kette der Formel $-[CH_2]_n-$ mit n = 3, 4, 5 oder 6 oder eine Kette der Formel $-[CH_2]_p-CH=CH-[CH_2]_q-$ mit (p+q) = 1, 2, 3 oder 4 bilden oder

c) A und C = Wasserstoff bedeuten und $B^1$ und $B^2$ gemeinsam eine Kette der Formel $-[CH_2]_m-$ mit m = 4, 5, 6 oder 7 bilden,

durch Kristallisation diastereomerer Salze, dadurch gekennzeichnet, dass man die Salze der racemischen Ester mit N-acylierten, optisch aktiven R- oder S-Aminocarbonsäuren, die einen Phenylkern enthalten und deren eventuell vorhandenen freien OH-Gruppen gegebenenfalls durch Alkyl mit 1 bis 6 C-Atomen, Benzyl oder andere in der Peptidchemie übliche OH-Schutzgruppen O-alkyliert sind, aus der Reihe Phenylalanin, C-Phenylglycin, β-Phenyl-α-amino-buttersäure, 3,4-Dihydroxyphenylalanin, β-Phenylserin und Tyrosin, herstellt, diese aus einem aprotischen organischen Lösemittel oder einem Alkohol mit bis zu 6 C-Atomen umkristallisiert, ausgefallene, optische einheitliche diastereomere Salze in an sich bekannter Weise zerlegt und die Enantiomeren der Formeln Ia bzw. Ib isoliert und diese gegebenenfalls durch Verseifung bzw. Hydrogenolyse in an sich bekannter Weise in die freien Säuren überführt.

2. Verfahren gemäss Anspruch 1, dadurch gekennzeichnet, dass

a) A und $B^1$ Wasserstoff bedeuten und $B^2$ und C gemeinsam eine Kette der Formel $-[CH_2]_n-$ mit n = 3, 4, 5, oder 6 oder eine Kette der Formel $-[CH_2]_p-CH=CH-[CH_2]_q-$ mit (p+q) = 1, 2, 3 oder 4 bilden oder

b) C und $B^2$ Wasserstoff bedeuten und A und $B^1$ gemeinsam eine der vorstehend unter a) definierten Ketten bilden.

3. Verfahren nach Anspruch 2, dadurch gekennzeichnet, dass man die Salze racemischer Ester der Formeln Ia bzw. Ib, in welchen die beiden Brückenkopf-Wasserstoffatome, cis-konfiguriert sind und die COOR-Gruppe endo-ständig zum bicyclischen Ringsystem orientiert ist, abscheidet.

4. Verfahren nach einem der Ansprüche 1 bis 3, dadurch gekennzeichnet, dass R für Alkyl mit 1 bis 6 C-Atomen, Cycloalkyl mit 4 bis 8 C-Atomen oder Aralkyl mit 7 bis 13 C-Atomen, das gegebenenfalls durch $NO_2$ substituiert sein kann, steht.

5. Verfahren nach einem der Ansprüche 1 bis 4, dadurch gekennzeichnet, dass die Aminofunktion der zu Salzbildung verwendeten N-acylierten R- oder S-Aminocarbonsäuren durch Alkanoyl mit 1 bis 6 C-Atomen, tert.-Butoxycarbonyl, Benzyloxycarbonyl oder eine andere, in der Peptidchemie übliche $NH_2$-Schutzgruppe geschützt ist und evtl. vorhandene freie OH-Funktionen der N-acylierten Aminosäuren gegebenenfalls durch Alkyl mit 1 bis 6 C-Atomen Benzyl oder andere, in der Peptidchemie übliche OH-Schutzgruppen geschützt sind.

6. Verbindung der Formeln Ia oder Ib, in welchen die beiden Brückenkopf-Wasserstoffatome cis-konfiguriert sind, die COOR-Gruppe endoständig zum bicyclischen Ringsystem orientiert ist, das zu COOR-Gruppe α-ständige C-Atom R- oder S-konfiguriert ist und A, $B^1$, $B^2$ und C die in Anspruch 2 und R die in Anspruch 4 definierten Bedeutungen haben, sowie deren Salze.

7. Verbindung der Formeln Ia oder Ib, in welchen die beiden Brückenkopf-Wasserstoffatome cis-konfiguriert sind, die COOR-Gruppe endoständig zum bicyclischen Ringsystem orientiert ist, das zu COOR-Gruppe α-ständige C-Atom R- oder S-konfiguriert ist und

R Wasserstoff bedeutet oder die in Anspruch 4 definierten Bedeutungen hat,

a) A und $B^1$ Wasserstoff bedeuten und $B^2$ und C gemeinsam eine Kette der Formel $-[CH_2]_n-$ mit n = 3, 4, 5 oder 6 oder eine Kette der Formel $-[CH_2]_p-CH=CH-[CH_2]_q-$ mit (p+q) = 1, 2, 3 oder 4 bilden oder

b) C und $B^2$ Wasserstoff bedeuten und A und $B^1$ gemeinsam eine der vorstehend unter a) definierten Ketten mit n = 3, 5 oder 6 bzw. (p+q) = 1, 2, 3 oder 4 bilden, sowie deren Salze.

8. Verbindung gemäss einem der Ansprüche 6 oder 7, dadurch gekennzeichnet, dass das zur COOR-Gruppe α-ständige C-Atom S-konfiguriert ist.

9. Diastereomeres Salz aus einem bicyclischen Imino-α-carbonsäureester der Formeln Ia oder Ib gemäss einem der Ansprüche 6 oder 8, in welchen R die in Anspruch 4 definierten Bedeutungen hat, und einer N-acylierten, optisch aktiven R- oder S-Aminocarbonsäure, die einen Phenylkern enthält und die, wie im Anspruch 5 definiert, geschützt ist.

10. cis, endo-2-Azabicyclo[3.3.0]octan-3S-carbonsäure.

11. cis, endo-2-Azabicyclo[3.3.0]octan-3S-carbonsäureester, in welchem R die im Anspruch 4 definierte Bedeutung hat.

12. cis, endo-2-Azabicyclo[3.3.0]octan-3S-carbonsäurebenzylester.

13. Verfahren gemäss einem der Ansprüche 1 bis 5 zur Abtrennung eines cis, endo-Octahydro-[1H]indol-2S-carbonsäureesters, in welchem R die in Anspruch 4 definierte Bedeutung hat.

14. Verfahren gemäss einem der Ansprüche 1 bis 5 zur Abtrennung von cis, endo-Octahydro-[1H]indol-2S-carbonsäurebenzylester.

15. Verfahren zur Herstellung von optisch reinen Verbindungen der allgemeinen Formel IIa oder IIb

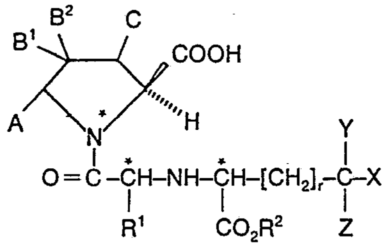

(IIa)

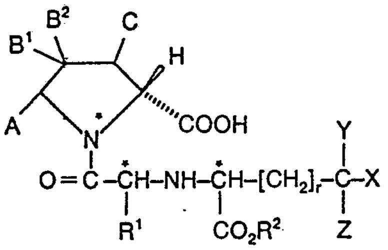

(IIb)

in welchen die mit einem Stern (*) markierten Kohlenstoffatome jeweils unabhängig voneinander die R- oder die S-Konfiguration aufweisen können,

a) A und $B^1$ = Wasserstoff bedeuten und

$B^2$ und C gemeinsam eine Kette der Formel $-[CH_2]_n-$ mit n = 3, 4, 5 oder 6 oder eine Kette der Formel $-[CH_2]_p-CH=CH-[CH_2]_q-$ mit (p+q) = 1, 2, 3 oder 4 bilden,

b) C und $B^2$ = Wasserstoff bedeuten und

A und $B^1$ gemeinsam eine Kette der Formel $-[CH_2]_n-$ mit n = 3, 4, 5 oder 6 oder eine Kette der Formel $-[CH_2]_p-CH=CH-[CH_2]_q-$ mit (p+q) = 1, 2, 3 oder 4 bilden oder

c) A und C = Wasserstoff bedeuten und

$B^1$ und $B^2$ gemeinsam eine Kette der Formel $-[CH_2]_m-$ mit m = 4, 5, 6 oder 7 bilden,

r = 0 oder 1,

$R^1$ = Wasserstoff, einen gegebenenfalls substituierten aliphatischen Rest mit 1 bis 6 C-Atomen, einen gegebenenfalls substituierten alicyclischen Rest mit 3 bis 9 C-Atomen, einen gegebenenfalls substituierten alicyclisch-aliphatischen Rest mit 4 bis 11 C-Atomen, einen gegebenenfalls substituierten aromatischen Rest mit 6 bis 12 C-Atomen, der auch teilhydriert sein kann, einen gegebenenfalls substituierten araliphatischen Rest mit 7 bis 15 C-Atomen, einen gegebenenfalls substituierten aroylaliphatischen Rest mit 8 bis 13 C-Atomen, einen gegebenenfalls substituierten mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer natürlich vorkommenden gegebenenfalls geschützten Aminosäure,

$R^2$ = Wasserstoff, einen gegebenenfalls substituierten aliphatischen Rest mit 1 bis 6 C-Atomen, substituierten araliphatischen Rest mit 7 bis 15 C-Atomen,

Y = Wasserstoff oder Hydroxy,

Z = Wasserstoff oder

Y und Z = zusammen Sauerstoff und

X = einen aliphatischen Rest mit 1 bis 6 C-Atomen, einen alicyclischen Rest mit 5 bis 9 C-Atomen, einen gegebenenfalls substituierten aromatischen Rest mit 6 bis 12 C-Atomen oder Indolyl bedeuten,

dadurch gekennzeichnet, dass man optisch reine Verbindungen der Formeln Ia oder Ib, in welchen A, $B^1$, $B^2$ und C die obengenannte Bedeutung besitzen und

R für einen gegebenenfalls substituierten aliphatischen Rest mit 1 bis 6 C-Atomen, einen gegebenenfalls substituierten alicyclischen Rest mit 4 bis 10 C-Atomen, einen gegebenenfalls substituierten aromatischen Rest mit 6 bis 12 C-Atomen oder einen gegebenenfalls substituierten araliphatischen Rest mit 7 bis 15 C-Atomen steht, mit optisch reinen Verbindungen der Formel III

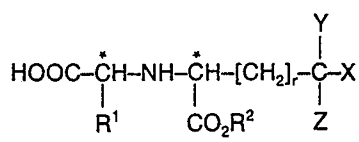

(III)

in der r, $R^1$, $R^2$, X, Y und Z die obengenannte Bedeutung besitzen, in Anwesenheit eines Kondensationsmittels oder gegebenenfalls als Aktivester umsetzt, den Rest R durch Hydrogenolyse oder Hydrolyse abspaltet und die optisch reinen Verbindungen der Formeln IIa oder IIb gegebenenfalls in physiologisch verträgliche Salze überführt.

16. Verfahren gemäss Anspruch 15, dadurch gekennzeichnet, dass Verbindungen der Formeln IIa oder IIb hergestellt werden, in denen

r = 0 oder 1,

R = Wasserstoff, ($C_1$ bis $C_6$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$)-Cycloalkyl-($C_1$ bis $C_4$)-alkyl, Aryl oder teilhydriertes Aryl mit 6 bis 12 C-Atomen, das jeweils durch ($C_1$ bis $C_4$)-Alkyl, ($C_1$ oder $C_2$)-Alkoxy oder Halogen substituiert sein kann, ($C_6$–$C_{12}$)-Aryl-($C_1$–$C_4$)-alkyl oder ($C_7$–$C_{13}$)-Aroyl-($C_1$–$C_2$)-alkyl, die beide wie vorstehend definiert im Arylrest substituiert sein können, einen mono- bzw. bicyclischen Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden Aminosäure,

$R^2$ = Wasserstoff, ($C_1$ bis $C_6$)-Alkyl, ($C_2$ bis $C_6$)-Alkenyl oder ($C_6$ bis $C_{12}$)-Aryl-($C_1$ bis $C_4$)-alkyl,

Y = Wasserstoff oder Hydroxy,

Z = Wasserstoff oder

Y und Z = zusammen Sauerstoff und

X = ($C_1$ bis $C_6$)-Alkyl, ($C_2$ bis $C_6$)-Alkenyl, ($C_5$ bis $C_9$)-Cycloalkyl, ($C_6$ bis $C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)-alkylamino und/oder Methylendioxy mono-, di- oder trisubstituiert sein kann, oder 3-Indolyl bedeuten.

17. Verfahren gemäss einem der Ansprüche 15 oder 16, dadurch gekennzeichnet, dass Verbindungen der Formel IIa oder IIb hergestellt werden in denen

a) A und $B^1$ = Wasserstoff bedeuten und

$B^2$ und C = gemeinsam eine der in Anspruch 15 (unter a) definierten Ketten bilden oder

b) C und $B^2$ = Wasserstoff bedeuten und

A und $B^1$ gemeinsam eine der im Anspruch 15 (unter b) definierten Ketten bilden.

18. Verfahren gemäss einem der Ansprüche 15–17, dadurch gekennzeichnet, dass die (S,S,S)-Verbindung hergestellt wird.

19. Verfahren gemäss Anspruch 17, dadurch gekennzeichnet, dass eine cis,endo-Verbindung hergestellt wird.

20. Verfahren gemäss einem der Ansprüche 1–5, dadurch gekennzeichnet, dass die erhaltenen Verbindungen der Formel Ia bzw. Ib nach einem Verfahren gemäss der Ansprüche 15–19 weiter umgesetzt werden.

**Patentansprüche für den Vertragsstaat AT**

1. Verfahren zur Auftrennung racemischer Gemische bicyclischer Imino-α-carbonsäureester in die Komponenten der Formel Ia und Ib

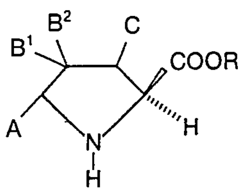

(Ia)

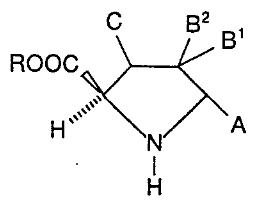

(Ib)

in welchen

R für einen aliphatischen Rest mit 1 bis 6 C-Atomen, einen alicyclischen Rest mit 4 bis 10 C-Atomen, einen aromatischen Rest mit 6 bis 12 C-Atomen oder einen araliphatischen Rest mit 7 bis 15 C-Atomen steht,

a) A und $B^1$ = Wasserstoff bedeuten und

$B^2$ und C gemeinsam eine Kette der Formel $-[CH_2]_n-$ mit n = 3, 4, 5 oder 6 oder eine Kette der Formel $-[CH_2]_p-CH=CH-[CH_2]_q-$ mit $(p+q) = 1$, 2, 3 oder 4 bilden,

b) C und $B^2$ = Wasserstoff bedeuten und

A und $B^1$ gemeinsam eine Kette der Formel $-[CH_2]_n-$ mit n = 3, 4, 5 oder 6 oder eine Kette der Formel $-[CH_2]_p-CH=CH-[CH_2]_q-$ mit $(p+q) = 1$, 2, 3 oder 4 bilden oder

c) A und C = Wasserstoff bedeuten und

$B^1$ und $B^2$ gemeinsam eine Kette der Formel $-[CH_2]_m-$ mit m = 4, 5, 6 oder 7 bilden,

durch Kristallisation diastereomerer Salze, dadurch gekennzeichnet, dass man die Salze der racemischen Ester mit N-acylierten, optisch aktiven R- oder S-Aminocarbonsäuren, die einen Phenylkern enthalten und deren eventuell vorhandenen freien OH-Gruppen gegebenenfalls durch Alkyl mit 1 bis 6 C-Atomen, Benzyl oder andere in der Peptidchemie übliche OH-Schutzgruppen O-alkyliert sind, aus der Reihe Phenylalanin, C-Phenylglycin, β-Phenyl-α-amino-buttersäure, 3,4-Dihydroxyphenylalanin, β-Phenylserin und Tyrosin, herstellt, diese aus einem aprotischen organischen Lösemittel oder einem Alkohol mit bis zu 6 C-Atomen umkristallisiert, ausgefallene, optisch einheitliche diastereomere Salze in an sich bekannter Weise zerlegt und die Enantiomeren der Formeln Ia bzw. Ib isoliert und diese gegebenenfalls durch Verseifung bzw. Hydrogenolyse in an sich bekannter Weise in die freien Säuren überführt.

2. Verfahren gemäss Anspruch 1, dadurch gekennzeichnet, dass

a) A und $B^1$ Wasserstoff bedeuten und

$B^2$ und C gemeinsam eine Kette der Formel $-[CH_2]_n-$ mit n = 3, 4, 5 oder 6 oder eine Kette der Formel $-[CH_2]_p-CH=CH-[CH_2]_q-$ mit $(p+q) = 1$, 2, 3 oder 4 bilden oder

b) C und $B^2$ Wasserstoff bedeuten und

A und $B^1$ gemeinsam eine der vorstehend unter a) definierten Ketten bilden.

3. Verfahren nach Anspruch 2, dadurch gekennzeichnet, dass man die Salze racemischer Ester der Formeln Ia bzw. Ib, in welchen die beiden Brückenkopf-Wasserstoffatome cis-konfiguriert sind und die COOR-Gruppe endo-ständig zum bicyclischen Ringsystem orientiert ist, abscheidet.

4. Verfahren nach einem der Ansprüche 1 bis 3, dadurch gekennzeichnet, dass R für Alkyl mit 1 bis 6 C-Atomen, Cycloalkyl mit 4 bis 8 C-Atomen oder Aralkyl mit 7 bis 13 C-Atomen, das gegebenenfalls durch $NO_2$ substituiert sein kann, steht.

5. Verfahren nach einem der Ansprüche 1 bis 4, dadurch gekennzeichnet, dass die Aminofunktion der zur Salzbildung verwendeten N-acylierten R- oder S-Aminocarbonsäuren durch Alkanoyl mit 1 bis 6 C-Atomen, tert.-Butoxycarbonyl, Benzyloxycarbonyl oder eine andere, in der Peptidchemie übliche $NH_2$-Schutzgruppe geschützt ist und evtl. vorhandene freie OH-Funktionen der N-acylierten Aminosäuren gegebenenfalls durch Alkyl mit 1 bis 6 C-Atomen Benzyl oder andere, in der Peptidchemie übliche OH-Schutzgruppen geschützt sind.

6. Verfahren gemäss Anspruch 1 zur Abtrennung einer Verbindung der Formeln Ia oder Ib, in welchen die beiden Brückenkopf-Wasserstoffatome cis-konfiguriert sind, die COOR-Gruppe endoständig zum bicyclischen Ringsystem orientiert ist, das zu COOR-Gruppe α-ständige C-Atom R- oder S-konfiguriert ist und A, $B^1$, $B^2$ und C die in Anspruch 2 und R die in Anspruch 4 definierten Bedeutungen haben, sowie deren Salze.

7. Verfahren gemäss Anspruch 1 zur Abtrennung einer Verbindung der Formeln Ia oder Ib, in welchen die beiden Brückenkopf-Wasserstoffatome cis-konfiguriert sind, die COOR-Gruppe endoständig zum bicyclischen Ringsystem orientiert ist, das zu COOR-Gruppe α-ständige C-Atom R- oder S-konfiguriert ist und

R Wasserstoff bedeutet oder die in Anspruch 4 definierten Bedeutungen hat,

a) A und $B^1$ Wasserstoff bedêuten und

$B^2$ und C gemeinsam eine Kette der Formel $-[CH_2]_n-$ mit n = 3, 4, 5 oder 6 oder eine Kette der

Formel $-[CH_2]_p-CH=CH-[CH_2]_q-$ mit $(p+q) = 1$, 2, 3 oder 4 bilden oder

b) C und $B^2$ Wasserstoff bedeuten und

A und $B^1$ gemeinsam eine der vorstehend unter a) definierten Ketten mit n = 3, 5 oder 6 bzw. $(p+q) = 1$, 2, 3 oder 4 bilden, sowie deren Salze.

8. Verfahren gemäss einem der Ansprüche 6 oder 7, dadurch gekennzeichnet, dass das zur COOR-Gruppe α-ständige C-Atom S-konfiguriert ist.

9. Verfahren gemäss Anspruch 1 zur Herstellung eines diastereomeren Salzes aus einem bicyclischen Imino-α-carbonsäureester der Formeln Ia oder Ib gemäss einem der Ansprüche 6 oder 8, in welchen R die in Anspruch 4 definierten Bedeutungen hat, und einer N-acylierten, optisch aktiven R- oder S-Aminocarbonsäure, die einen Phenylkern enthält und die, wie im Anspruch 5 definiert, geschützt ist.

10. Verfahren gemäss Anspruch 7 zur Abtrennung von cis,endo-2-Azabicyclo[3.3.0]octan-3S-carbonsäure.

11. Verfahren gemäss Anspruch 6 oder 7 zur Abtrennung eines cis,endo-2-Azabicyclo[3.3.0]octan-3S-carbonsäureesters, in welchem R die im Anspruch 4 definierte Bedeutung hat.

12. Verfahren gemäss Anspruch 6, 7 oder 11 zur Abtrennung von cis,endo-2-Azabicyclo[3.3.0]octan-3S-carbonsäurebenzylester.

13. Verfahren gemäss Anspruch 6 oder 7 zur Abtrennung eines cis,endo-Octahydro[1H]indol-2S-carbonsäureesters, in welchem R die in Anspruch 4 definierte Bedeutung hat.

14. Verfahren gemäss Anspruch 6, 7 oder 13 zur Abtrennung von cis,endo-Octahydro[1H]indol-2S-carbonsäurebenzylester.

15. Verfahren zur Herstellung von optisch reinen Verbindungen der allgemeinen Formeln IIa oder IIb

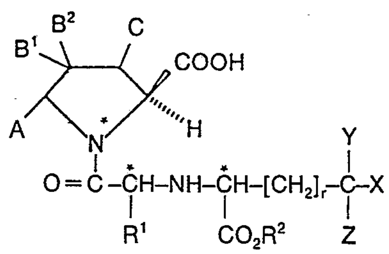

(IIa)

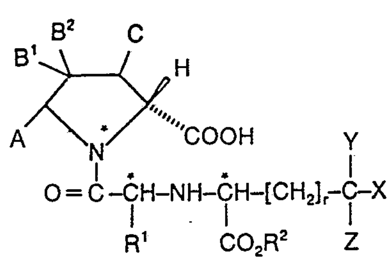

(IIb)

in welchen die mit einem Stern (*) markierten Kohlenstoffatome jeweils unabhängig voneinander die R- oder die S-Konfiguration aufweisen können,

a) A und $B^1$ = Wasserstoff bedeuten und

$B^2$ und C gemeinsam eine Kette der Formel $-[CH_2]_n-$ mit n = 3, 4, 5 oder 6 oder eine Kette der Formel $-[CH_2]_p-CH=CH-[CH_2]_q-$ mit $(p+q) = 1$, 2, 3 oder 4 bilden,

b) C und $B^2$ = Wasserstoff bedeuten und

A und $B^1$ gemeinsam eine Kette der Formel $-[CH_2]_n-$ mit n = 3, 4, 5 oder 6 oder eine Kette der Formel $-[CH_2]_p-CH=CH-[CH_2]_q-$ mit $(p+q) = 1$, 2, 3 oder 4 bilden oder

c) A und C = Wasserstoff bedeuten und

$B^1$ und $B^2$ gemeinsam eine Kette der Formel $-[CH_2]_m-$ mit m = 4, 5, 6 oder 7 bilden,

r = 0 oder 1,

$R^1$ = Wasserstoff, einen gegebenenfalls substituierten aliphatischen Rest mit 1 bis 6 C-Atomen, einen gegebenenfalls substituierten alicyclischen Rest mit 3 bis 9 C-Atomen, einen gegebenenfalls substituierten alicyclisch-aliphatischen Rest mit 4 bis 11 C-Atomen, einen gegebenenfalls substituierten aromatischen Rest mit 6 bis 12 C-Atomen, der auch teilhydriert sein kann, einen gegebenenfalls substituierten araliphatischen Rest mit 7 bis 15 C-Atomen, einen gegebenenfalls substituierten aroylaliphatischen Rest mit 8 bis 13 C-Atomen, einen gegenenenfalls substituierten mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer natürlich vorkommenden gegebenenfalls geschützten Aminosäure,

$R^2$ = Wasserstoff, einen gegebenenfalls substituierten aliphatischen Rest mit 1 bis 6 C-Atomen, substituierten araliphatischen Rest mit 7 bis 15 C-Atomen,

Y = Wasserstoff oder Hydroxy,

Z = Wasserstoff oder

Y und Z = zusammen Sauerstoff und

X = einen aliphatischen Rest mit 1 bis 6 C-Atomen, einen alicyclischen Rest mit 5 bis 9 C-Atomen, einen gegebenenfalls substituierten aromatischen Rest mit 6 bis 12 C-Atomen oder Indolyl bedeuten,

dadurch gekennzeichnet, dass man optisch reine Verbindungen der Formeln Ia oder Ib, in welchen A, $B^1$, $B^2$ und C die obengenannte Bedeutung besitzen und

R für einen gegebenenfalls substituierten aliphatischen Rest mit 1 bis 6 C-Atomen, einen gegebenenfalls substituierten alicyclischen Rest mit 4 bis 10 C-Atomen, einen gegebenenfalls substituierten aromatischen Rest mit 6 bis 12 C-Atomen oder einen gegebenenfalls substituierten araliphatischen Rest mit 7 bis 15 C-Atomen steht, mit optisch reinen Verbindungen der Formel III

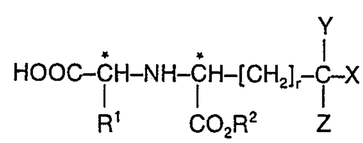

(III)

in der r, $R^1$, $R^2$, X, Y und Z die obengenannte Bedeutung besitzen, in Anwesenheit eines Kondensationsmittels oder gegebenenfalls als Aktivester umsetzt, den Rest R durch Hydrogenolyse oder Hydrolyse abspaltet und die optisch reinen

Verbindungen der Formeln IIa und IIb gegebenenfalls in physiologisch verträgliche Salze überführt.

16. Verfahren gemäss Anspruch 15, dadurch gekennzeichnet, dass Verbindungen der Formeln IIa oder IIb hergestellt werden, in denen

r = 0 oder 1,

R = Wasserstoff, ($C_1$ bis $C_6$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$)-Cycloalkyl-($C_1$ bis $C_4$)-alkyl, Aryl oder teilhydriertes Aryl mit 6 bis 12 C-Atomen, das jeweils durch ($C_1$ bis $C_4$)-Alkyl, ($C_1$ oder $C_2$)-Alkoxy oder Halogen substituiert sein kann, ($C_6$–$C_{12}$-Aryl-($C_1$–$C_4$)-alkyl oder ($C_7$–$C_{13}$)-Aroyl-($C_1$–$C_2$)-alkyl, die beide wie vorstehend definiert im Arylrest substituiert sein können, einen mono- bzw. bicyclischen Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden Aminosäure,

$R^2$ = Wasserstoff, ($C_1$ bis $C_6$)-Alkyl, ($C_2$ bis $C_6$)-Alkenyl oder ($C_6$ bis $C_{12}$)-Aryl-($C_1$ bis $C_4$)-alkyl,

Y = Wasserstoff oder Hydroxy,

Z = Wasserstoff oder

Y und Z = zusammen Sauerstoff und

X = ($C_1$ bis $C_6$)-Alkyl, ($C_2$ bis $C_6$)-Alkenyl, ($C_5$ bis $C_9$)-Cycloalkyl, ($C_6$ bis $C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)-alkylamino und/oder Methylendioxy mono-, di- oder trisubstituiert sein kann, oder 3-Indolyl bedeuten.

17. Verfahren gemäss einem der Ansprüche 15 oder 16, dadurch gekennzeichnet, dass Verbindungen der Formel IIa oder IIb hergestellt werden in denen

a) A und $B^1$ = Wasserstoff bedeuten und

$B^2$ und C gemeinsam eine der in Anspruch 15 (unter a) definierten Ketten bilden oder

b) C und $B^2$ = Wasserstoff bedeuten und

A und $B^1$ gemeinsam eine der im Anspruch 15 (unter b) definierten Ketten bilden.

18. Verfahren gemäss einem der Ansprüche 15–17, dadurch gekennzeichnet, dass die (S,S,S)-Verbindung hergestellt wird.

19. Verfahren gemäss Anspruch 17, dadurch gekennzeichnet, dass eine cis,endo-Verbindung hergestellt wird.

20. Verfahren gemäss einem der Ansprüche 1–5, dadurch gekennzeichnet, dass die erhaltenen Verbindungen der Formel Ia bzw. Ib nach einem Verfahren gemäss der Ansprüche 15–19 weiter umgesetzt werden.

**Claims for the Contracting States BE CH DE FR GB IT LI LU NL SE**

1. A process for resolving racemic mixtures of bicyclic imino-α-carboxylic esters into the components of the formula Ia and Ib

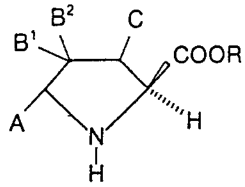

(Ia)

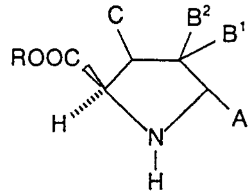

(Ib)

in which

R represents an aliphatic radical having 1 to 6 carbon atoms, an alicyclic radical having 4 to 10 carbon atoms, an aromatic radical having 6 to 12 carbon atoms or an araliphatic radical having 7 to 15 carbon atoms,

a) A and $B^1$ denote hydrogen, and

$B^2$ and C together form a chain of the formula $-[CH_2]_n-$, with n being 3, 4, 5 or 6, or a chain of the formula $-[CH_2]_p-CH=CH-[CH_2]_q-$, with (p+q) being 1, 2, 3 or 4,

b) C and $B^2$ denote hydrogen, and

A and $B^1$ together form a chain of the formula $-[CH_2]_n-$, with n being 3, 4, 5 or 6, or a chain of the formula $-[CH_2]_p-CH=CH-[CH_2]_q-$, with (p+q) being 1, 2, 3 or 4, or

c) A and C denote hydrogen, and

$B^1$ and $B^2$ together form a chain of the formula $-[CH_2]_m-$, with m being 4, 5, 6 or 7,

by crystallization of diastereomeric salts, which process comprises preparing the salts of the racemic esters with optically active N-acylated R- or S-aminocarboxylic acids which contain a phenyl nucleus, from the series phenylalanine, phenylglycine, β-phenyl-α-aminobutyric acid, 3,4-dihydroxyphenylalanin, β-phenylserine and tyrosine, recrystallizing them form an aprotic organic solvent or an alcohol having up to 6 carbon atoms, decomposing the precipitated, optically homogenous diastereomeric salts in a manner known per se, and isolating the enantiomers of the formula Ia or Ib, respectively, and where appropriate, converting the latter into the free acids by saponification or hydrogenolysis in a manner known per se.

2. The process as claimed in claim 1, wherein

a) A and $B^1$ denote hydrogen, and

$B^2$ and C together form a chain of the formula $-[CH_2]_n-$ with n being 3, 4, 5 or 6, or a chain of the formula $-[CH_2]_p-CH=CH-[CH_2]_q-$ with (p+q) being 1, 2, 3 or 4, or

b) C and $B^2$ denote hydrogen, and

A and $B^1$ together form one of the chains defined above under a).

3. The process as claimed in claim 2, wherein the salts of racemic esters of the formulae Ia and Ib in which the two bridgehead hydrogen atoms have the cis configuration and the COOR group is oriented endo with respect to the bicyclic ring system are precipitated.

4. The process as claimed in one of claims 1 to 3, wherein R represents alkyl having 1 to 6 carbon atoms, cycloalkyl having 4 to 8 carbon atoms or aralkyl having 7 to 13 carbon atoms which can optionally be substituted by $NO_2$.

5. The process as claimed in one of claims 1 to 4, wherein the amino group of the N-acylated R- or S-aminocarboxylic acids which are used for salt formation is protected by alkanoyl having 1 to 6 carbon atoms, tert.-butoxycarbonyl, benzyloxycarbonyl or another $NH_2$ protective group customary in peptide chemistry, and any free OH groups present in the N-acylated aminoacids are, where appropriate, protected by alkyl having 1 to 6 carbon atoms, benzyl or other OH protective groups customary in peptide chemistry.

6. A compound of the formulae Ia or Ib in which the two bridgehead hydrogen atoms have the cis configuration, the COOR group is oriented endo with respect to the bicyclic ring system, the carbon atom α to the COOR group has the R or S configuration, and A, $B^1$, $B^2$ and C have the meanings defined in claim 2, and R has that defined in claim 4, and its salts.

7. A compound of the formulae Ia or Ib in which the two bridgehead hydrogen atoms have the cis configuration, the COOR group is oriented endo with respect to the bicyclic ring system, the carbon atom α to the COOR group has the R or S configuration, and R denotes hydrogen or has the meanings defined in claim 4,

a) A and $B^1$ denote hydrogen, and

$B^2$ and C together form a chain of the formula $-[CH_2]_n-$, with n being 3, 4, 5 or 6, or a chain of the formula $-[CH_2]_p-CH=CH-[CH_2]_q-$, with (p+q) being 1, 2, 3 or 4, or

b) C and $B^2$ denote hydrogen, and

A and $B^1$ together form one of the chains defined above a), with n being 3, 5 or 6 and (p+q) being 1, 2, 3 or 4, and its salts.

8. A compound as claimed in one of claims 6 or 7, wherein the carbon atom α to the COOR group has the S configuration.

9. A diastereomeric salt of bicyclic imino-α-carboxylic ester of the formulae Ia or Ib as claimed in one of claims 6 or 8, in which R has the meanings defined in claim 4, and an N-acylated, optically active R- or S-aminocarboxylic acid which contains a phenyl nucleus and is protected as defined in claim 5.

10. cis,endo-2-Azabicyclo[3.3.0]octane-3S-carboxylic acid.

11. A cis,endo-2-azabicyclo[3.3.0]octane-3S-carboxylic ester in which R has the meaning defined in claim 4.

12. Benzyl cis,endo-2-azabicyclo[3.3.0]octane-3S-carboxylate.

13. A process as claimed in one of claims 1 to 4 for separating off benzyl cis,endo-octahydro[1H]indole-2S-carboxylate.

14. A process as claimed in one of claims 1 to 4 for separating off benzyl cis, endo-octahydro[1H]indole-2S-carboxylate.

15. A process for the preparation of optically pure compounds of the general formulae IIa or IIb

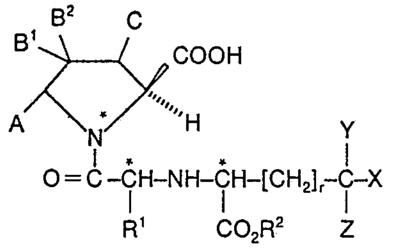

(IIa)

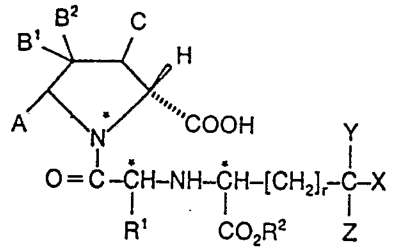

(IIb)

in which it is possible for the carbon atoms labeled with an asterisk (*) each, independently of one another, to have the R- or the S-configuration,

a) A and $B^1$ denote hydrogen, and

$B^2$ and C together form a chain of the formula $-[CH_2]_n-$, with n being 3, 4, 5 or 6, or a chain of the formula $-[CH_2]_p-CH=CH-[CH_2]_q-$, with (p+q) being 1, 2, 3 or 4,

b) C and $B^2$ denote hydrogen, and

A and $B^1$ together form a chain of the formula $-[CH_2]_n-$, with n being 3, 4, 5 or 6, or a chain of the formula $-[CH_2]_p-CH=CH-[CH_2]_p-$, with (p+q) being 1, 2, 3 or 4, or

c) A and C denote hydrogen, and

$B^1$ and $B^2$ together form a chain of the formula $-[CH_2]_m-$, with m being 4, 5, 6 or 7,

r denotes 0 or 1,

$R^1$ denotes hydrogen, an optionally substituted aliphatic radical having 1 to 6 carbon atoms, an optionally substituted alicyclic radical having 3 to 9 carbon atoms, an optionally substituted alicyclic-aliphatic radical having 4 to 11 carbon atoms, an optionally substituted aromatic radical having 6 to 12 carbon atoms, which can also be partially hydrogenated, an optionally substituted araliphatic radical having 7 to 15 carbon atoms, an optionally substituted aroyl-aliphatic radical having 8 to 13 carbon atoms, an optionally substituted monocyclic or bicyclic heterocyclic radical having 5 to 7 or 8 to 10 ring atoms respectively, 1 or 2 of these ring atoms being sulfur or oxygen atoms and/or 1 to 4 of these ring atoms being nitrogen atoms, or a side chain of a naturally occurring aminoacid which is optionally protected,

$R^2$ denotes hydrogen, an optionally substituted aliphatic radical having 1 to 6 carbon atoms, or an optionally substituted araliphatic radical having 7 to 15 carbon atoms,

Y denotes hydrogen or hydroxyl,

Z denotes hydrogen, or

Y and Z together denote oxygen, and

X denotes an aliphatic radical having 1 to 6 carbon atoms, an alicyclic radical having 5 to 9 carbon atoms, an optionally substituted aromatic radical having 6 to 12 carbon atoms or indolyl, which process comprises reacting, in the

presence of a condensing agent or, where appropriate, as an active ester, optically pure compounds of the formulae Ia or Ib, in which A, $B^1$, $B^2$ and C have the abovementioned meanings, and

R represents an optionally substituted aliphatic radical having 1 to 6 carbon atoms, an optionally substituted alicyclic radical having 4 to 10 carbon atoms, an optionally substituted aromatic radical having 6 to 12 carbon atoms or an optionally substituted araliphatic radical having 7 to 15 carbon atoms, with optically pure compounds of the formula III

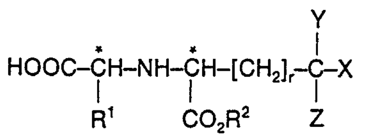

(III)

in which r, $R^1$, $R^2$, X, Y and Z have the abovementioned meanings splitting off the radical R by hydrogenolysis or hydrolysis, and, where appropriate, converting the optically pure compounds of the formulae IIa or IIb into physiologically tolerated salts.

16. The process as claimed in claim 15, wherein are prepared compounds with the formulae IIa or IIb in which

r denotes 0 or 1,

R denotes hydrogen, ($C_1$ to $C_6$)-alkyl or aralkyl having 7 to 9 carbon atoms,

$R^1$ denotes hydrogen or ($C_1$ to $C_6$)-alkyl, which can optionally be substituted by amino, ($C_1$ to $C_6$)-acyl-amino or benzoylamino, ($C_2$ to $C_6$)-alkenyl, ($C_5$ to $C_9$)-cycloalkyl, ($C_5$ to $C_9$)-cycloalkenyl, ($C_5$ to $C_7$)-cycloalkyl-($C_1$ to $C_4$)-alkyl, aryl or partially hydrogenated aryl having 6 to 12 carbon atoms, each of which can be substituted by ($C_1$ to $C_4$)-alkyl, ($C_1$ to $C_2$)-alkoxy or halogen, ($C_6$ to $C_{12}$)-aryl-($C_1$–$C_4$)-alkyl or ($C_7$–$C_{13}$)-aroyl-($C_1$–$C_2$)-alkyl, both of which can be substituted in the aryl radical as defined above, a monocyclic or bicyclic heterocyclic radical having 5 to 7 or 8 to 10 ring atoms respectively, 1 or 2 of these ring atoms being sulfur or oxygen atoms and/or 1 to 4 of these ring atoms being nitrogen atoms, or an optionally protected side chain of a naturally occurring aminoacid,

$R_2$ denotes hydrogen, ($C_1$ to $C_6$)-alkyl, ($C_2$ to $C_6$)-alkenyl or ($C_6$ to $C_{12}$)-aryl-($C_1$ to $C_4$)-alkyl,

Y denotes hydrogen or hydroxyl,

Z denotes hydrogen, or

Y and Z together denote oxygen, and

X denotes ($C_1$ to $C_6$)-alkyl, ($C_2$ to $C_6$)-alkenyl, ($C_5$ to $C_9$)-cycloalkyl, ($C_6$ to $C_{12}$)-aryl, which can be monosubstituted, disubstituted or trisubstituted by ($C_1$ to $C_4$)-alkyl, ($C_1$ to $C_4$)-alkoxy, hydroxyl, halogen, nitro, amino, ($C_1$ to $C_4$)-alkylamino, di-($C_1$ to $C_4$)-alkylamino and/or methylenedioxy, or 3-imdolyl.

17. The process as claimed in one of claims 15 or 16, wherein are prepared compounds of the formula IIa or IIb in which

a) A and $B^1$ denote hydrogen, and

$B^2$ and C together form one of the chains defined in claim 15 (under a) or

b) C and $B^2$ denote hydrogen, and

A and $B^1$ together form one of the chains defined in claim 15 (under b).

18. The process as claimed in one of claims 15–17, wherein the (S,S,S) compound is prepared.

19. The process as claimed in claim 17, wherein a cis,endo compound is prepared.

20. The process as claimed in one of claims 1–5, wherein the resulting compounds of the formula Ia or Ib are further reacted by a process according to claims 15–19.

**Claims for the Contracting State AT**

1. A process for resolving racemic mixtures of bicyclic imino-α-carboxylic esters into the components of the formula Ia and Ib

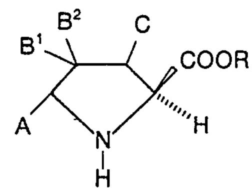

(Ia)

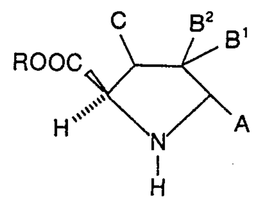

(Ib)

in which

R represents an aliphatic radical having 1 to 6 carbon atoms, an alicyclic radical having 4 to 10 carbon atoms, an aromatic radical having 6 to 12 carbon atoms or an araliphatic radical having 7 to 15 carbon atoms,

a) A and $B^1$ denote hydrogen, and

$B^2$ and C together form a chain of the formula $-[CH_2]_n-$, with n being 3, 4, 5 or 6, or a chain of the formula $-[CH_2]_p-CH=CH-[CH_2]_q-$, with (p+q) being 1, 2, 3 or 4,

b) C and $B^2$ denote hydrogen, and

A and $B^1$ together form a chain of the formula $-[CH_2]_n-$, with n being 3, 4, 5 or 6, or a chain of the formula $-[CH_2]_p-CH=CH-[CH_2]_q-$, with (p+q) being 1, 2, 3 or 4, or

c) A and C denote hydrogen, and

$B^1$ and $B^2$ together form a chain of the formula $-[CH_2]_m-$, with m being 4, 5, 6 or 7,

by crystallization of diastereomeric salts, which process comprises preparing the salts of the racemic esters with optically active N-acylated R- or S-aminocarboxylic acids which contain a phenyl nucleus, from the series phenylalanine, phenylglycine, β-phenyl-α-aminobutyric acid, 3,4-dihydroxyphenylalanin, β-phenylserine and tyrosine, recrystallizing them form an aprotic organic solvent or an alcohol having up to 6 carbon atoms, decomposing the precipitated, optically homogenous diastereomeric salts in a manner known per se, and isolating the enantiomers of the formulae Ia or Ib, respectively, and, where appropriate, converting the latter into the free acids by saponification or hydrogenolysis in a manner known per se.

2. The process as claimed in claim 1, wherein

a) A and $B^1$ denote hydrogen, and

$B^2$ and C together form a chain of the formula $-[CH_2]_n-$, with n being 3, 4, 5 or 6, or a chain of the

formula $-[CH_2]_p-CH=CH-[CH_2]_q-$ with $(p+q)$ being 1, 2, 3 or 4, or

b) C and $B^2$ denote hydrogen, and

A and $B^1$ together form one of the chains defined above under a).

3. The process as claimed in claim 2, wherein the salts of racemic esters of the formulae Ia and Ib in which the two bridgehead hydrogen atoms have the cis configuration and the COOR group is oriented endo with respect to the bicyclic ring system are precipitated.

4. The process as claimed in one of claims 1 to 3, wherein R represents alkyl having 1 to 6 carbon atoms, cycloalkyl having 4 to 8 carbon atoms or aralkyl having 7 to 13 carbon atoms which can optionally be substituted by $NO_2$.

5. The process as claimed in one of claims 1 to 4, wherein the amino group of the N-acylated R- or S-aminocarboxylic acids which are used for salt formation is protected by alkanoyl having to 6 carbon atoms, tert.-butoxycarbonyl, benzyloxycarbonyl or another $NH_2$ protective group customary in peptide chemistry, any free OH groups present in the N-acylated aminoacids are, where appropriate, protected by alkyl having 1 to 6 carbon atoms, benzyl or other OH protective groups customary in peptide chemistry.

6. The process as claimed in claim 1 for separating off a compound of the formulae Ia or Ib in which the two bridgehead hydrogen atoms have the cis configuration, the COOR group is oriented endo with respect to the bicyclic ring system, the carbon atom $\alpha$ to the COOR group has the R or S configuration, and A, $B^1$, $B^2$ and C have the meanings defined in claim 2, and R has the defined in claim 4, and its salts.

7. The process as claimed in claim 1 for separating off a compound of the formulae Ia or Ib in which the two bridgehead hydrogen atoms have the cis configuration, the COOR group is oriented endo with respect to the bicyclic ring system, the carbon atom $\alpha$ to the COOR group has the R or S configuration, and R denotes hydrogen or has the meanings defined in claim 4,

a) A and $B^1$ denote hydrogen, and

$B^2$ and C together form a chain of the formula $-[CH_2]_n-$, with n being 3, 4, 5 or 6, or a chain of the formula $-[CH_2]_p-CH=CH-[CH_2]_q-$, with $(p+q)$ being 1, 2, 3 or 4, or

b) C and $B^2$ denote hydrogen, and

A and $B^1$ together form one of the chains defined above a), with n being 3, 5 or 6 and $(p+q)$ being 1, 2, 3 or 4, and its salts.

8. The process as claimed in one of claims 6 or 7, characterized in that the carbon atom $\alpha$ to the COOR group has the S configuration.

9. The process as claimed in claim 1 for the preparation of diastereomeric salt of a bicyclic imino-$\alpha$-carboxylic ester of the formulae Ia or Ib as claimed in one of claims 6 or 8, in which R has the meanings defined in claim 4, and an N-acylated, optically active R- or S-aminocarboxylic acid which contains a phenyl nucleus and is protected as defined in claim 5.

10. The process as claimed in claim 7 for separating off cis,endo-2-azabicyclo[3.3.0]octane-3S-carboxylic acid.

11. The process as claimed in claim 6 or 7 for separating off cis,endo-2-azabicyclo[3.3.0]octane-3S-carboxylic ester in which R has the meaning defined in claim 4.

12. The process as claimed in claim 6, 7 or 11 for separating off benzyl cis,endo-2-azabicyclo[3.3.0]octane-3S-carboxylate.

13. The process as claimed in claim 6 or 7 for separating off process as claimed in one of claims 1 to 4 for separating off benzyl cis,endo-octahydro[1H]indole-2S-carboxylate.

14. The process as claimed in claims 6, 7 or 13 for separating off benzyl cis,endo-octahydro[1H]indole-2S-carboxylate.

15. A process for the preparation of optically pure compounds of the general formulae IIa or IIb

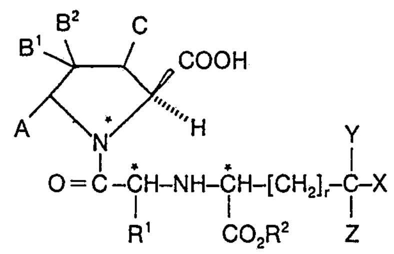

(IIa)

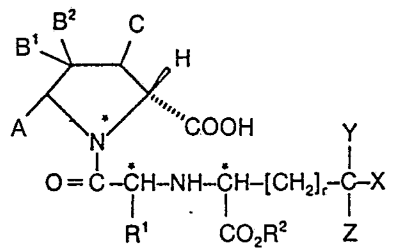

(IIb)

in which it is possible for the carbon atoms labeled with an asterisk (*) each, independently of one another, to have the R- or the S-configuration,

a) A and $B^1$ denote hydrogen, and

$B^2$ and C together form a chain of the formula $-[CH_2]_n-$, with n being 3, 4, 5 or 6, or a chain of the formula $-[CH_2]_p-CH=CH-[CH_2]_q-$, with $(p+q)$ being 1, 2, 3 or 4,

b) C and $B^2$ denote hydrogen, and

A and $B^1$ together form a chain of the formula $-[CH_2]_n-$, with n being 3, 4, 5 or 6, or a chain of the formula $-[CH_2]_p-CH=CH-[CH_2]_q-$, with $(p+q)$ being 1, 2, 3 or 4, or

c) A and C denote hydrogen, and

$B^1$ and $B^2$ together form a chain of the formula $-[CH_2]_m-$, with m being 4, 5, 6 or 7,

r denotes 0 or 1,

$R^1$ denotes hydrogen, an optionally substituted aliphatic radical having 1 to 6 carbon atoms, an optionally substituted alicyclic radical having 3 to 9 carbon atoms, an optionally substituted alicyclic-aliphatic radical having 4 to 11 carbon atoms, an optionally substituted aromatic radical having 6 to 12 carbon atoms, which can also be partially hydrogenated, an optionally substituted araliphatic radical having 7 to 15 carbon atoms, an optionally

substituted aroyl-aliphatic radical having 8 to 13 carbon atoms, an optionally substituted monocyclic or bicyclic heterocyclic radical having 5 to 7 or 8 to 10 ring atoms respectively, 1 or 2 of these ring atoms being sulfur or oxygen atoms and/or 1 to 4 of these ring atoms being nitrogen atoms, or a side chain of a naturally occurring aminoacid which is optionally protected,

$R^2$ denotes hydrogen, an optionally substituted aliphatic radical having 1 to 6 carbon atoms, or an optionally substituted araliphatic radical having 7 to 15 carbon atoms,

Y denotes hydrogen or hydroxyl,

Z denotes hydrogen, or

Y and Z together denote oxygen, and

X denotes an aliphatic radical having 1 to 6 carbon atoms, an alicyclic radical having 5 to 9 carbon atoms, an optionally substituted aromatic radical having 6 to 12 carbon atoms, or indolyl, which process comprises reacting, in the presence of a condensing agent or, where appropriate, as an active ester, optically pure compounds of the formulae Ia or Ib, in which A, $B^1$, $B^2$ and C have the abovementioned meanings, and

R represents an optionally substituted aliphatic radical having 1 to 6 carbon atoms, an optionally substituted alicyclic radical having 4 to 10 carbon atoms, an optionally substituted aromatic radical having 6 to 12 carbon atoms or an optionally substituted araliphatic radical having 7 to 15 carbon atoms, with optically pure compounds of the formula III

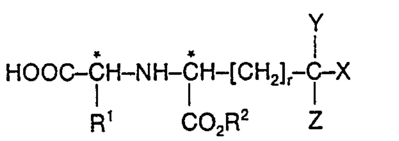

(III)

in which r, $R^1$, $R^2$, X, Y and Z have the abovementioned meanings splitting off the radical R by hydrogenolysis or hydrolysis, and, where appropriate, converting the optically pure compounds of the formulae IIa or IIb into physiologically tolerated salts.

16. The process as claimed in claim 15, wherein are prepared compounds with the formulae IIa or IIb in which

r denotes 0 or 1,

R denotes hydrogen, ($C_1$ to $C_6$)-alkyl or aralkyl having 7 to 9 carbon atoms,

$R^1$ denotes hydrogen or ($C_1$ to $C_6$)-alkyl, which can optionally be substituted by amino, ($C_1$ to $C_6$)-acylamino or benzoylamino, ($C_2$ to $C_6$)-alkenyl, ($C_5$ to $C_9$)-cycloalkyl, ($C_5$ to $C_9$)-cycloalkenyl, ($C_5$ to $C_7$)-cycloalkyl-($C_1$ to $C_4$)-alkyl, aryl or partially hydrogenated aryl having 6 to 12 carbon atoms, each of which can be substituted by ($C_1$ to $C_4$)-alkyl, ($C_1$ to $C_2$)-alkoxy or halogen, ($C_6$ to $C_{12}$)-aryl-($C_1$–$C_4$)-alkyl or ($C_7$–$C_{13}$)-aroyl-($C_1$–$C_2$)-alkyl, both of which can be substituted in the aryl radical as defined above, a monocyclic or bicyclic heterocyclic radical having 5 to 7 or 8 to 10 ring atoms respectively, 1 or 2 of these ring atoms being sulfur or oxygen atoms and/or 1 to 4 of these ring atoms being nitrogen atoms, or an optionally protected side chain of a naturally occurring aminoacid,

$R^2$ denotes hydrogen, ($C_1$ to $C_6$)-alkyl, ($C_2$ to $C_6$)-alkenyl or ($C_6$ to $C_{12}$)-aryl-($C_1$ to $C_4$)-alkyl,

Y denotes hydrogen or hydroxyl,

Z denotes hydrogen, or

Y and Z together denote oxygen, and

X denotes ($C_1$ to $C_6$)-alkyl, ($C_2$ to $C_6$)-alkenyl, ($C_5$ to $C_9$)-cycloalkyl, ($C_6$ to $C_{12}$)-aryl, which can be monosubstituted, disubstituted or trisubstituted by ($C_1$ to $C_4$)-alkyl, ($C_1$ to $C_4$)-alkoxy, hydroxyl, halogen, nitro, amino, ($C_1$ to $C_4$)-alkylamino, di-($C_1$ to $C_4$)-alkylamino and/or methylenedioxy, or 3-indolyl.

17. The process as claimed in one of claims 15 or 16, wherein are prepared compounds of the formula IIa or IIb in which

a) A and $B^1$ denote hydrogen, and

$B^2$ and C together form one of the chains defined in claim 15 (under a) or

b) C and $B^2$ denote hydrogen, and

A and $B^1$ together form one of the chains defined in claim 15 (under b).

18. The process as claimed in one of claims 15–17, wherein the (S,S,S) compound is prepared.

19. The process as claimed in claim 17, wherein a cis,endo compound is prepared.

20. The process as claimed in one of claims 1–5, wherein the resulting compounds of the formula Ia or Ib are further reacted by a process according to claims 15–19.

**Revendications pour les Etats Contractants BE CH DE FR GB IT LT LU NL SE**

1. Procédé de séparation de mélanges racémiques d'esters d'acides imino-α-carboxyliques bicycliques en les constituants de formules Ia et Ib

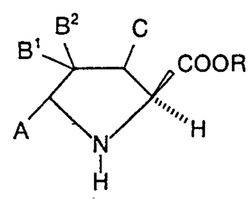

(Ia)

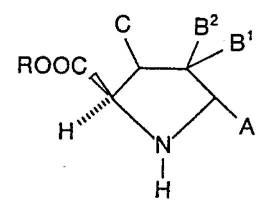

(Ib)

dans lesquelles

R représente un groupe aliphatique ayant de 1 à 6 atomes de carbone, un groupe alicyclique ayant de 4 à 10 atomes de carbone, un groupe aromatique ayant de 6 à 12 atomes de carbone ou un groupe araliphatique ayant de 7 à 15 atomes de carbone,

a) A et $B^1$ représentent l'hydrogène et

$B^2$ et C forment ensemble une chaîne de formule $-(CH_2)_n-$ avec n = 3, 4, 5 ou 6 ou une chaîne de formule $-(CH_2)_p-CH=CH-(CH_2)_q-$ avec (p+q) = 1, 2, 3 ou 4,

b) C et $B^2$ représentent l'hydrogène et

A et $B^1$ forment ensemble une chaîne de formule $-(CH_2)_n-$ avec n = 3, 4, 5 ou 6, ou une chaîne

de formule $-(CH_2)_p-CH=CH-(CH_2)_q-$ avec $(p+q) = 1, 2, 3$ ou 4 ou

c) A et C représentent l'hydrogène et

$B^1$ et $B^2$ forment ensemble une chaîne de formule $-(CH_2)_m-$ avec $m = 4, 5, 6$ ou 7,

par cristallisation de sels diastéréomères, ce procédé étant caractérisé en ce qu'on prépare les sels des esters racémiques avec des acides R- ou S-aminocarboxyliques optiquement actifs N-acylés qui contiennent un noyau phényle et dont les groupes OH libres éventuellement présents sont éventuellement O-alcoylés par des groupes alkyle de 1 à 6 atomes de carbone, benzyle ou autres groupes protecteurs d'OH habituels dans la chimie des peptides, de la série de la phénylalanine, de la C-phénylglycine, de l'acide -phényl-α-amino-butyrique, de la 3,4-dihydroxyphénylalanine, de la β-phénylsérine et de la tyrosine, on cristallise ces sels dans un solvant organique aprotique ou dans un alcool ayant jusqu'à 6 atomes de carbone, on décompose d'une manière connue en soi les sels diastéréomères précipités optiquement homogènes et on isole les énantiomères de formule Ia ou Ib et éventuellement on convertit ces derniers d'une manière connue en soi en les acides libres, par saponification ou hydrogénolyse.

2. Procédé selon la revendication 1, caractérisé en ce que:

a) A et $B^1$ représentent l'hydrogène et

$B^2$ et C forment ensemble une chaîne de formule $-(CH_2)_n-$ avec $n = 3, 4, 5$ ou 6 ou une chaîne de formule $-(CH_2)_p-CH=CH-(CH_2)_q-$ avec $(p+q) = 1, 2, 3$ ou 4 ou

b) C et $B^2$ représentent l'hydrogène et

A et $B^1$ forment ensemble une des chaînes définies en a) ci-dessus.

3. Procédé selon la revendication 2, caractérisé en ce qu'on sépare les sels des esters racémiques de formule Ia ou Ib, dans lesquels les deux atomes d'hydrogène têtes de pont sont en configuration cis et le groupe COOR a une orientation endo par rapport au système bicyclique.

4. Procédé selon l'une des revendications 1 à 3, caractérisé en ce que R représente un groupe alcoyle ayant de 1 à 6 atomes de carbone, cycloalcoyle ayant de 4 à 8 atomes de carbone ou aralcoyle ayant de 7 à 13 atomes de carbone, qui peut être éventuellement substitué par $NO_2$.

5. Procédé selon l'une des revendications 1 à 4, caractérisé en ce que la fonction amino des acides R- ou S-aminocarboxyliques N-acylés utilisés pour la formation du sel, est protégée par un alcanoyl ayant de 1 à 6 atomes de carbone, un groupe t-butoxy-carbonyle, benzyloxycarbonyle ou un autre groupe $NH_2-$ protecteur usuel dans la chimie des peptides, et les fonctions OH libres, éventuellement présentes, des acides aminés N-acylés, sont protégés le cas échéant par un groupe alcoyle ayant de 1 à 6 atomes de carbone, benzyle ou par d'autres groupes OH protecteurs usuels dans la chimie des peptides.

6. Composé de formule Ia ou Ib, dans lequel les deux atomes d'hydrogène têtes de pont sont en configuration cis, les groupes COOR a une orientation endo par rapport au système bicyclique, l'atome de carbone en position α par rapport au groupe COOR a une configuration R ou S et A, $B^1$, $B^2$ et C ont les significations définies dans la revendication 2 et R a la signification définie dans la revendication 4, ainsi que ses sels.

7. Composé de formule Ia ou Ib, dans lequel les deux atomes d'hydrogène têtes de pont sont en configuration cis, le groupe COOR a une orientation endo par rapport au système bicyclique, l'atome de carbone en position α par rapport au groupe COOR, a une configuration R ou S et

R représente l'hydrogène ou a l'une des significations indiquées dans la revendication 4,

a) A et $B^1$ représentent l'hydrogène et

$B^2$ et C forment ensemble une chaîne de formule $-(CH_2)_n-$ avec $n = 3, 4, 5$ ou 6 ou une chaîne de formule $-(CH_2)_p-CH=CH-(CH_2)_q-$ avec $(p+q) = 1, 2, 3$ ou 4 ou

b) C et $B^2$ représentent l'hydrogène et

A et $B^1$ forment ensemble une des chaînes définies en a) ci-dessus, avec $n = 3, 5$ ou 6 ou $(p+q) = 1, 2, 3$ ou 4, ainsi que ses sels.

8. Composé selon la revendication 6 ou 7, caractérisé en ce que l'atome de carbone en position α par rapport au groupe COOR a la configuration S.

9. Sel diastéréomère d'un ester d'acide imino-α-carboxylique bicyclique de formule Ia ou Ib selon la revendication 6 ou 8, dans lequel R a les significations définies dans la revendication 4, et d'un acide R ou S-aminocarboxylique optiquement actif, N-acylé, qui contient un noyau phényle, et qui est protégé comme défini dans la revendication 5.

10. Acide cis, endo-2-azabicyclo[3.3.0]-octane-3S-carboxylique.

11. Ester de l'acide cis, endo-2-azabicyclo-[3.3.0]-octane-3S-carboxylique, dans lequel R a la signification définie dans la revendication 4.

12. Ester benzilique de l'acide cis, endo-2-azabicyclo[3.3.0]-octane-3S-carboxylique.

13. Procédé selon l'une des revendications 1 à 5 pour la séparation d'un ester de l'acide cis, endo-octahydro[1H]indole-2S-carboxylique, dans lequel R a la signification définie dans la revendication 4.

14. Procédé selon l'une des revendications 1 à 5 pour la séparation d'un ester benzylique de l'acide cis, endo-octahydro[1H]indole-2S-carboxylique.

15. Procédé de préparation de composés optiquement purs de formule générale IIa ou IIb

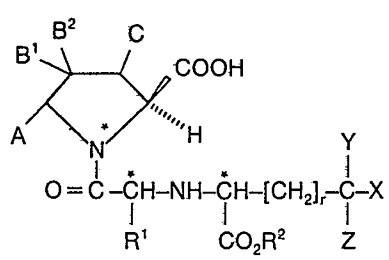

(IIa)

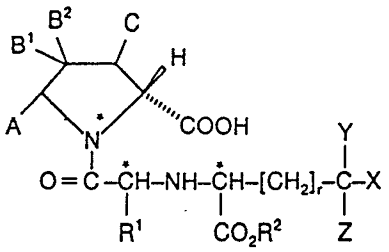

(IIb)

dans lesquelles les atomes de carbones marqués d'un astérisque (*) peuvent montrer la configuration R ou S indépendamment les uns des autres.

a) A et $B^1$ représentent l'hydrogène et

$B^2$ et C forment ensemble une chaîne de formule $-(CH_2)_n-$ avec n = 3, 4, 5 ou 6 ou une chaîne de formule $-(CH_2)_p-CH=CH-(CH_2)_q-$ avec (p+q) = 1, 2, 3 ou 4,

b) C et $B^2$ représentent l'hydrogène et

A et $B^1$ forment ensemble une chaîne de formule $-(CH_2)_n-$ avec n = 3, 4, 5 ou 6 ou une chaîne de formule $-(CH_2)_p-CH=CH-(CH_2)_q-$ avec (p+q) = 1, 2, 3 ou 4 ou

c) A et C représentent l'hydrogène et

$B^1$ et $B^2$ forment ensemble une chaîne de formule $-(CH_2)_m-$ avec m = 4, 5, 6 ou 7,

r = 0 ou 1,

$R^1$ représente l'hydrogène, un groupe aliphatique éventuellement substitué ayant de 1 à 6 atomes de carbone, un groupe alicyclique éventuellement substitué ayant de 3 à 9 atomes de carbone, un groupe alicyclique-aliphatique éventuellement substitué ayant de 4 à 11 atomes de carbone, un groupe aromatique éventuellement substitué ayant de 6 à 12 atomes de carbone, qui peut être également partiellement hydrogéné, un groupe araliphatique éventuellement substitué ayant de 7 à 15 atomes de carbone, un groupe aroylaliphatique éventuellement substitué ayant de 8 à 13 atomes de carbone, un groupe hétérocyclique mono- ou bicyclique éventuellement substitué ayant de 5 à 7 ou de 8 à 10 atomes dans le noyau, dont 1 à 2 des atomes dans le noyau représentent des atomes de soufre ou d'oxygène et/ou dont de 1 à 4 des atomes dans le noyau représentent des atomes d'azote, ou une chaîne latérale d'un acide aminé naturel, éventuellement protégé,

$R^2$ représente l'hydrogène, un groupe aliphatique éventuellement substitué ayant de 1 à 6 atomes de carbone, un groupe araliphatique éventuellement substitué ayant de 7 à 15 atomes de carbone,

Y représente l'hydrogène ou un groupe hydroxy,

Z représente l'hydrogène ou

Y et Z représentent ensemble l'oxygène et

X représente un groupe aliphatique ayant de 1 à 6 atomes de carbone, un groupe alicyclique ayant de 5 à 9 atomes de carbone, un groupe aromatique éventuellement substitué ayant de 6 à 12 atomes de carbone ou un groupe indolyle,

ce procédé étant caractérisé en ce qu'on fait réagir des composés optiquement purs de formule Ia ou Ib dans lesquels A, $B^1$, $B^2$ et C ont les significations indiquées ci-dessus et

R représente un groupe aliphatique éventuellement substitué ayant de 1 à 6 atomes de carbone, un groupe alicyclique éventuellement substitué ayant de 4 à 10 atomes de carbone, un groupe aromatique éventuellement substitué ayant de 6 à 12 atomes de carbone ou un groupe araliphatique éventuellement substitué ayant de 7 à 15 atomes de carbone,

avec des composés optiquement purs de formule III

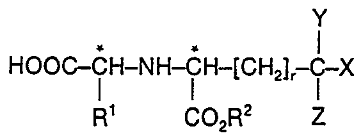

(III)

dans laquelle r, $R^1$, $R^2$, X, Y et Z ont les significations indiquées ci-dessus, en présence d'un agent de condensation ou éventuellement sous la forme d'un ester actif, on élimine le groupe R par hydrogénolyse ou hydrolyse et on convertit éventuellement les composés optiquement purs de formule IIa ou IIb en des sels physiologiquement acceptables.

16. Procédé selon la revendication 15, caractérisé en ce qu'on prépare des composés de formule IIa ou IIb, dans lesquels:

r et 0 ou 1,

R représente l'hydrogène, un groupe alcoyle en $C_1$–$C_6$ ou aralcoyle ayant de 7 à 9 atomes de carbone,

$R^1$ représente l'hydrogène ou un groupe alcoyle en $C_1$–$C_6$, qui peut être éventuellement substitué par un groupe amino, acyl($C_1$–$C_6$)-amino ou benzoylamino; un groupe alcényle en $C_2$–$C_6$, cycloalcoyle en $C_5$–$C_9$, cycloalcényle en $C_5$–$C_9$, cycloalcoyl ($C_5$–$C_7$)-alcoyle en $C_1$–$C_4$, aryle ou aryle partiellement hydrogéné ayant de 6 à 12 atomes de carbone, qui peuvent être chacun substitués par un groupe alcoyle en $C_1$–$C_4$, alcoxy en $C_1$ ou $C_2$ ou par un halogène; un groupe aryl ($C_6$–$C_{12}$)-alcoyle en $C_1$–$C_4$ ou aroyl ($C_7$–$C_{13}$)-alcoyle en $C_1$–$C_2$, qui peuvent être substitués tous les deux dans le groupe aryle comme défini précédemment; un groupe hétérocyclique mono- ou bicyclique ayant de 5 à 7 ou de 8 à 10 atomes dans le noyau, dont de 1 à 2 des atomes du noyau représentent des atomes de soufre ou d'oxygène et/ou de 1 à 4 des atomes du noyau représentent des atomes d'azote; ou une chaîne latérale éventuellement protégée d'un acide aminé naturel,

$R^2$ représente l'hydrogène, un groupe alcoyle en $C_1$–$C_6$, alcényle en $C_2$–$C_6$ ou aryl ($C_6$–$C_{12}$)-alcoyle en $C_1$–$C_4$,

Y représente l'hydrogène ou un groupe hydroxy,

Z représente l'hydrogène ou

Y et Z représentent ensemble l'oxygène et

X représente un groupe alcoyle en $C_1$–$C_6$, alcényle en $C_2$–$C_6$, cycloalcoyle en $C_5$–$C_9$, aryle en $C_6$–$C_{12}$, qui peut être mono-, di-, ou trisubstitué par un groupe alcoyle en $C_1$–$C_4$, alcoxy en $C_1$–$C_4$, hydroxy, un halogène, un groupe nitro, amino, al-

coyl-($C_1$–$C_4$)-amino, di-alcoyl($C_1$–$C_4$)-amino et/ou méthylènedioxy; ou un groupe 3-indolyle.

17. Procédé selon la revendication 15 ou 16, caractérisé en ce qu'on prépare des composés de formule IIa ou IIb, dans lesquels:

a) A et $B^1$ représentent l'hydrogène et

$B_2$ et C forment ensemble une des chaînes définies dans la revendication 15 (en a) ou

b) C et $B^2$ représentent l'hydrogène et

A et $B^1$ forment ensemble une des chaînes définies dans la revendication 15 (en b).

18. Procédé selon l'une des revendications 15 à 17, caractérisé en ce qu'on prépare le composé (S,S,S).

19. Procédé selon la revendication 17, caractérisé en ce qu'on prépare un composé cis, endo.

20. Procédé selon l'une des revendications 1 à 5, caractérisé en ce qu'on fait ensuite réagir les composés de formule Ia ou Ib obtenus dans un procédé selon l'une quelconque des revendications 15 à 19.

**Revendications pour l'Etat Contractant AT**

1. Procédé de séparation de mélanges racémiques d'esters d'acides imino-α-carboxyliques bicycliques en les constituants de formules Ia et Ib

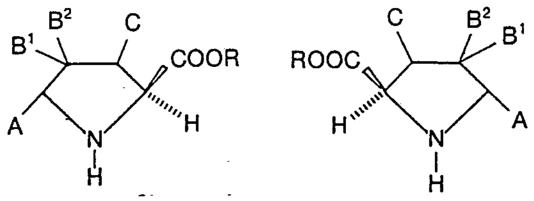

(Ia) (Ib)

dans lesquelles

R représente un groupe aliphatique ayant de 1 à 6 atomes de carbone, un groupe alicyclique ayant de 4 à 10 atomes de carbone, un groupe aromatique ayant de 6 à 12 atomes de carbone ou un groupe araliphatique ayant de 7 à 15 atomes de carbone,

a) A et $B^1$ représentent l'hydrogène et

$B^2$ et C forment ensemble une chaîne de formule $-(CH_2)_n-$ avec n = 3, 4, 5 ou 6 ou une chaîne de formule $-(CH_2)_p-CH=CH-(CH_2)_q-$ avec (p+q) = 1, 2, 3 ou 4,

b) C et $B^2$ représentent l'hydrogène et

A et $B^1$ forment ensemble une chaîne de formule $-(CH_2)_n-$ avec n = 3, 4, 5 ou 6, ou une chaîne de formule $-(CH_2)_p-CH=CH-(CH_2)_q-$ avec (p+q) = 1, 2, 3 ou 4 ou

c) A et C représentent l'hydrogène et

$B^1$ et $B^2$ forment ensemble une chaîne de formule $-(CH_2)_m-$ avec m = 4, 5, 6 ou 7,

par cristallisation de sels diastéréomères, ce procédé étant caractérisé en ce qu'on prépare les sels des esters racémiques avec des acides R- ou S-aminocarboxyliques optiquement actifs N-acylés qui contiennent un noyau phényle et dont les groupes OH libres éventuellement présents sont éventuellement O-alcoylés par des groupes alkyle de 1 à 6 atomes de carbone, benzyle ou autres groupes protecteurs d'OH habituels dans la chimie des peptides, de la série de la phénylalanine, de la C-phénylglycine, de l'acide β-phényl-α-amino-butyrique, de la 3,4-dihyroxyphénylalanine, de la β-phénylsérine et de la tyrosine, on cristallise ces sels dans un solvant organique aprotique ou dans un alcool ayant jusqu'à 6 atomes de carbone, on décompose d'une manière connue en soi les sels diastéréomères précipités optiquement homogènes et on isole les énantiomères de formule Ia ou Ib et éventuellement on convertit ces derniers d'une manière connue en soi en les acides libres, par saponification ou hydrogénolyse.

2. Procédé selon la revendication 1, caractérisé en ce que:

a) A et $B^1$ représentent l'hydrogène et

$B^2$ et C forment ensemble une chaîne de formule $-(CH_2)_n-$ avec n = 3, 4, 5 ou 6 ou une chaîne de formule $-(CH_2)_p-CH=CH-(CH_2)_q-$ avec (p+q) = 1, 2, 3 ou 4 ou

b) C et $B^2$ représentent l'hydrogène et

A et $B^1$ forment ensemble une des chaînes définies en a) ci-dessus.

3. Procédé selon la revendication 2, caractérisé en ce qu'on sépare les sels des esters racémiques de formule Ia ou Ib, dans lesquels les deux atomes d'hydrogène têtes de pont sont en configuration cis et le groupe COOR a une orientation endo par rapport au système bicyclique.

4. Procédé selon l'une des revendications 1 à 3, caractérisé en ce que R représente un groupe alcoyle ayant de 1 à 6 atomes de carbone, cycloalcoyle ayant de 4 à 8 atomes de carbone ou aralcoyle ayant de 7 à 13 atomes de carbone, qui peut être éventuellement substitué par $NO_2$.

5. Procédé selon l'une des revendications 1 à 4, caractérisé en ce que la fonction amino des acides R- ou S-aminocarboxyliques N-acylés utilisés pour la formation du sel, est protégée par un alcanoyl ayant de 1 à 6 atomes de carbone, un groupe t-butoxy-carbonyle, benzyloxycarbonyle ou un autre groupe $NH_2$-protecteur usuel dans la chimie des peptides, et les fonctions OH libres, éventuellement présentes, des acides aminés N-acylés, sont protégés le cas échéant par un groupe alcoyle ayant de 1 à 6 atomes de carbone, benzyle ou par d'autres groupes OH protecteurs usuels dans la chimie des peptides.

6. Procédé selon la revendication 1 pour la séparation d'un composé de formule Ia ou Ib, dans lequel les deux atomes d'hydrogène têtes de pont sont en configuration cis, le groupe COOR a une orientation endo par rapport au système bicyclique, l'atome de carbone en position α par rapport au groupe COOR a une configuration R ou S et A, $B^1$, $B^2$ et C ont les significations définies dans la revendication 2 et R a la signification définie dans la revendication 4, ainsi que ses sels.

7. Procédé suivant la revendication 1 pour la séparation d'un composé de formule Ia ou Ib, dans lequel les deux atomes d'hydrogène têtes de pont sont en configuration cis, le groupe COOR a une orientation endo par rapport au système bicyclique, l'atome de carbone en position α par rapport au groupe COOR, a une configuration R ou S et

R représente l'hydrogène ou a l'une des significations indiquée dans la revendication 4,

a) A et $B^1$ représentent l'hydrogène et

$B^2$ et C forment ensemble une chaîne de formule $-(CH_2)_n-$ avec n = 3, 4, 5 ou 6 ou une chaîne de formule $-(CH_2)_p-CH=CH-(CH_2)_q-$ avec (p+q) = 1, 2, 3 ou 4 ou

b) C et $B^2$ représentent l'hydrogène et

A et $B^1$ forment ensemble une des chaînes définies en a) ci-dessus, avec n = 3, 5 ou 6 (p+q) = 1, 2, 3 ou 4, ainsi que ses sels.

8. Procédé selon la revendication 6 ou 7, caractérisé en ce que l'atome de carbone en position α par rapport au groupe COOR a la configuration S.

9. Procédé selon la revendication 1 pour la préparation d'un sel diastéréomère d'un ester d'acide imino-α-carboxylique bicyclique de formule Ia ou Ib selon la revendication 6 ou 8, dans lequel R a les significations définies dans la revendication 4, et d'un acide R- ou S-aminocarboxylique optiquement actif, N-acylé, qui contient un noyau phényle, et qui est protégé comme défini dans la revendication 5.

10. Procédé suivant la revendication 7 pour la séparation de l'acide cis, endo-2-azabicyclo-[3.3.0]-octane-3S-carboxylique.

11. Procédé selon la revendication 6 ou 7 pour la séparation d'un ester de l'acide cis, endo-2-azabicyclo[3.3.0]octane-3S-carboxylique, dans lequel R a la signification définie dans la revendication 4.

12. Procédé selon la revendication 6, 7 ou 11 pour la séparation de l'ester benzylique de l'acide cis, endo-2-azabicyclo[3.3.0]-octane-3S-carboxylique.

13. Procédé selon la revendication 6 ou 7 pour la séparation d'un ester de l'acide cis, endo-octahydro-[1H]indole-2S-carboxylique, dans lequel R a la signification définie dans la revendication 4.

14. Procédé selon la revendication 6, 7 ou 13 pour la séparation de l'ester benzylique de l'acide cis, endo-octahydro[1H]indole-2S-carboxylique.

15. Procédé de préparation de composés optiquement purs de formule générale IIa ou IIb

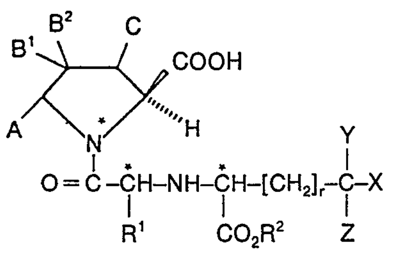

(IIa)

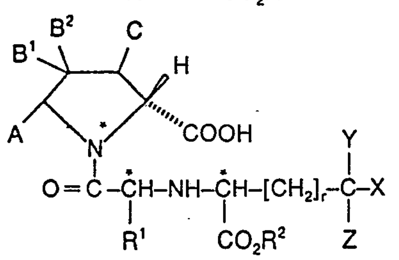

(IIb)

dans lesquelles les atomes de carbone marqués d'un astérisque (*) peuvent montrer la configuration R ou S indépendamment les uns des autres.

a) A et $B^1$ représentent l'hydrogène et

$B^2$ et C forment ensemble une chaîne de formule $-(CH_2)_n-$ avec n = 3, 4, 5 ou 6 ou une chaîne de formule $-(CH_2)_p-CH=CH-(CH_2)_q-$ avec (p+q) = 1, 2, 3 ou 4,

b) C et $B^2$ représentent l'hydrogène et

A et $B^1$ forment ensemble une chaîne de formule $-(CH_2)_n-$ avec n = 3, 4, 5 ou 6 ou une chaîne de formule $-(CH_2)_p-CH=CH-(CH_2)_q-$ avec (p+q) = 1, 2, 3 ou 4 ou

c) A et C représentent l'hydrogène et

$B^1$ et $C^2$ forment ensemble une chaîne de formule $-(CH_2)_m-$ avec m = 4, 5, 6 ou 7, r = 0 ou 1,

$R^1$ représente l'hydrogène, un groupe aliphatique éventuellement substitué ayant de 1 à 6 atomes de carbone, un groupe alicyclique éventuellement substitué ayant de 3 à 9 atomes de carbone, un groupe alicyclique-aliphatique éventuellement substitué ayant de 4 à 11 atomes de carbone, un groupe aromatique éventuellement substitué ayant de 6 à 12 atomes de carbone, qui peut être également partiellement hydrogéné, un groupe araliphatique éventuellement substitué ayant de 7 à 15 atomes de carbone, un groupe aroylaliphatique éventuellement substitué ayant de 8 à 13 atomes de carbone, un groupe hétérocyclique mono- ou bicyclique éventuellement substitué ayant de 5 à 7 ou de 8 à 10 atomes dans le noyau, dont 1 à 2 des atomes dans le noyau représentent des atomes de soufre ou d'oxygène et/ou dont de 1 à 4 des atomes dans le noyau représentent des atomes d'azote, ou une chaîne latérale d'un acide aminé naturel, éventuellement protégé,

$R^2$ représente l'hydrogène, un groupe aliphatique éventuellement substitué ayant de 1 à 6 atomes de carbone, un groupe araliphatique éventuellement substitué ayant de 7 à 15 atomes de carbone,

Y représente l'hydrogène ou un groupe hydroxy,

Z représente l'hydrogène ou

Y et Z représentent ensemble l'oxygène et

X représente un groupe aliphatique ayant de 1 à 6 atomes de carbone, un groupe alicyclique ayant de 5 à 9 atomes de carbone, un groupe aromatique éventuellement substitué ayant de 6 à 12 atomes de carbone ou un groupe indolyle, ce procédé étant caractérisé en ce qu'on fait réagir des composés optiquement purs de formule Ia ou Ib dans lesquels A, $B^1$ $B^2$ et C ont les significations indiquées ci-dessus et

R représente un groupe aliphatique éventuellement substitué ayant de 1 à 6 atomes de carbone, un groupe alicyclique éventuellement substitué ayant de 4 à 10 atomes de carbone, un groupe aromatique éventuellement substitué ayant de 6 à 12 atomes de carbone ou un groupe araliphatique éventuellement substitué ayant de 7 à 15 atomes de carbone,

avec des composés optiquement purs de formule III

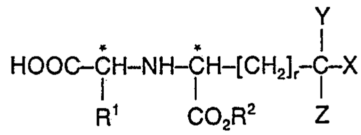

(III)

dans laquelle r, $R^1$, $R^2$, X, Y et Z ont les significations indiquées ci-dessus, en présence d'un agent de condensation ou éventuellement sous la forme d'un ester actif, on élimine le groupe R par hydrogénolyse ou hydrolyse et on convertit éventuellement les composés optiquement purs de formule IIa ou IIb en des sels physiologiquement acceptables.

16. Procédé selon la revendication 15, caractérisé en ce qu'on prépare des composés de formule IIa ou IIb, dans lesquels:

r est 0 ou 1,

R représente l'hydrogène, un groupe alcoyle en $C_1$–$C_6$ ou aralcoyle ayant de 7 à 9 atomes de carbone,

$R^1$ représente l'hydrogène ou un groupe alcoyle en $C_1$–$C_6$, qui peut être éventuellement substitué par un groupe amino, acyl($C_1$–$C_6$)-amino ou benzoylamino; un groupe alcényle en $C_2$–$C_6$, cycloalcoyle en $C_5$–$C_9$, cycloalcényle en $C_5$–$C_9$, cycloalcoyl ($C_5$–$C_7$)-alcoyle en $C_1$–$C_4$, aryle ou aryle partiellement hydrogéné ayant de 6 à 12 atomes de carbone, qui peuvent être chacun substitués par un groupe alcoyle en $C_1$–$C_4$, alcoxy en $C_1$ ou $C_2$ ou par un halogène; un groupe aryl ($C_6$–$C_{12}$)-alcoyle en $C_1$–$C_4$ ou aroyl ($C_7$–$C_{13}$)-alcoyle en $C_1$–$C_2$, qui peuvent être substitués tous les deux dans le groupe aryle comme défini précédemment; un groupe hétérocyclique mono- ou bicyclique ayant de 5 à 7 ou de 8 à 10 atomes dans le noyau, dont de 1 à 2 des atomes du noyau représentent des atomes de soufre ou d'oxygène et/ou de 1 à 4 des atomes du noyau représentent des atomes d'azote; ou une chaîne latérale éventuellement protégée d'un acide aminé naturel,

$R^2$ représente l'hydrogène, un groupe alcoyle en $C_1$–$C_6$, alcényle en $C_2$–$C_6$ ou aryl ($C_6$–$C_{12}$)-alcoyle en $C_1$–$C_4$,

Y représente l'hydrogène ou un groupe hydroxy,

Z représente l'hydrogène ou

Y et Z représentent ensemble l'oxygène et

X représente un groupe alcoyle en $C_1$–$C_6$, alcényle en $C_2$–$C_6$, cycloalcoyle en $C_5$–$C_9$, aryle en $C_6$–$C_{12}$, qui peut être mono-, di- ou trisubstitué par un groupe alcoyle en $C_1$–$C_4$, alcoxy en $C_1$–$C_4$, hydroxy, un halogène, un groupe nitro, amino, alcoyl-($C_1$–$C_4$)-amino, di-alcoyl($C_1$–$C_4$)-amino et/ou méthylènedioxy; ou un groupe 3-indolyle.

17. Procédé selon la revendication 15 ou 16, caractérisé en ce qu'on prépare des composés de formule IIa ou IIb, dans lesquels:

a) A et $B^1$ représentent l'hydrogène et

$B^2$ et C forment ensemble une des chaînes définies dans la revendication 15 (en a) ou

b) C et $B^2$ représentent l'hydrogène et

A et $B^1$ forment ensemble une des chaînes définies dans la revendication 15 (en b).

18. Procédé selon l'une des revendications 15 à 17, caractérisé en ce qu'on prépare le composé (S,S,S).

19. Procédé selon la revendication 17, caractérisé en ce qu'on prépare un composé cis, endo.

20. Procédé selon l'une des revendications 1 à 5, caractérisé en ce qu'on fait ensuite réagir les composés de formule Ia ou Ib obtenus dans un procédé selon l'une quelconque des revendications 15 à 19.